# EUROPEAN PATENT APPLICATION

(11) **EP 3 753 550 A1**
(43) Date of publication of application: **23.12.2020**
(21) Application number: 19750424.4
(22) Date of filing: 12.02.2019
(51) Int. Cl.: A61K 9/16, A61K 48/00, A61P 7/04

(54) **COMPOSITION FOR INCREASING EXPRESSION OF BLOOD COAGULATION FACTOR GENE, COMPRISING CORE-SHELL STRUCTURED MICROPARTICLES AS ACTIVE INGREDIENT**

(30) Priority: 12.02.2018 KR 20180017075
(71) Applicant: G&P Bioscience Co., Ltd., Seoul 07528 (KR); Reyon Pharmaceutical Co., Ltd., Gangnam-gu Seoul 06176 (KR)
(72) Inventor: HO, Seong-Hyun, Seoul 08052 (KR); PARK, Su Jin, Gwangmyeong-si Gyeonggi-do 14256 (KR)
(74) Representative: V.O.
(86) International application number: PCT/KR2019/001709
(87) International publication number: WO 2019/156541

(57) **Abstract**

The present disclosure relates to a composition for increasing the expression of a blood coagulation factor gene, which contains core-shell structured microparticles as an active ingredient. When administered *in vivo* along with blood coagulation factor VIII gene or a variant gene thereof, the composition for increasing the expression of a blood coagulation factor gene of the present disclosure can increase the expression of the gene by at least 30%. When administered along with a gene therapeutic agent, the composition can achieve a therapeutic effect even with a very small amount of a gene, and thus is useful.

## Description

### [Technical Field]

The present disclosure relates to a composition for increasing the expression of a blood coagulation factor gene, which contains core-shell structured microparticles as an active ingredient.

### [Background Art]

Hemophilia is one of severe genetic disorders. Hemophilia A is an X gene-linked disease occurring in 1 in 5000 males. It arises from the mutation of human blood coagulation factor VIII (also known as factor VIII, factor 8, FVIII or F8), which is an important plasma glycoprotein in blood clotting.

Before the 1980s, patients with hemophilia received administration of blood coagulation factor VIII extracted from the plasma of other people for treatment. But, this method had severe problems such as viral infection, etc. In order to overcome this advantage, full-length factor VIII produced in CHO cells, etc. based on recombinant protein researches are used for treatment since the 1980s.

The inventors of the present disclosure have researched to develop a gene therapeutic agent capable of achieving therapeutic effect even with a small amount of a gene. In doing so, they have identified that, when core-shell structured microparticles consisting of a halogenated hydrocarbon and/or halogenated sulfur as a core and a lipid component as an outer shell are administered *in vivo* along with a blood coagulation factor VIII gene, the expression of the blood coagulation factor gene is increased remarkably, and have completed the present disclosure.

### [References of Related Art]

### [Patent Documents]

(Patent document 001) KR 10-1542752 B.
(Patent document 002) KR 10-2018-0118659 A.

### [Disclosure]

### [Technical Problem]

The present disclosure is directed to providing a composition for increasing the expression of a blood coagulation factor gene, which contains core-shell structured microparticles as an active ingredient.

The present disclosure is also directed to providing a pharmaceutical composition for preventing or treating a bleeding disorder or bleeding, which contains the composition described above.

### [Technical Solution]

The present disclosure provides a composition for increasing the expression of a blood coagulation factor gene, which contains core-shell structured microparticles as an active ingredient, wherein the core is a halogenated hydrocarbon, halogenated sulfur or a mixture thereof as a biocompatible gas, and the shell is a lipid or a derivative thereof, and the blood coagulation factor gene is one or more gene selected from a human blood coagulation factor VIII gene or a variant gene thereof.

In an exemplary embodiment of the present disclosure, the biocompatible gas may be selected from sulfur hexafluoride, octafluoropropane, bromochlorodifluoromethane, chlorodifluoromethane, dichlorodifluoromethane, bromotrifluoromethane, chlorotrifluoromethane, chloropentafluoroethane, dichlorotetrafluoroethane and a mixture thereof.

In an exemplary embodiment of the present disclosure, the halogenated hydrocarbon may be a perfluorinated hydrocarbon.

In an exemplary embodiment of the present disclosure, the perfluorinated hydrocarbon may be perfluoromethane, perfluoroethane, perfluoropropane, perfluorobutane, perfluoropentane, perfluorohexane, perfluoroheptane, perfluoropropene, perfluorobutene, perfluorobutadiene, perfluorobut-2-ene, perfluorocyclobutane, perfluoromethylcyclobutane, perfluorodimethylcyclobutane, perfluorotrimethylcyclobutane, perfluorocyclopentane, perfluoromethylcyclopentane, perfluorodimethylcyclopentane, perfluoromethylcyclohexane, perfluoromethylcyclohexane, perfluoromethylcyclohexane or a mixture thereof.

In an exemplary embodiment of the present disclosure, the lipid may be one or more selected from a group consisting of a simple lipid, a phospholipid, a glyceroglycolipid, a sphingoglycolipid, a cholesterol and a cationic lipid.

In an exemplary embodiment of the present disclosure, the phospholipid may be selected from a group consisting of a phosphatidylcholine derivative, a phosphatidylethanolamine derivative, a phosphatidylserine derivative, a diacetylated phospholipid, L-α-dioleyl phosphatidylethanolamine, diolein, phosphatidic acid, phosphatidylglycerol, phosphatidylinositol, lysophosphatidylcholine, sphingomyelin, a polyethylene glycolated phospholipid, egg yolk lecithin, soy lecithin and a hydrogenated phospholipid.

In an exemplary embodiment of the present disclosure, the glyceroglycolipid may be selected from a group consisting of sulfoxyribosyl glyceride, diglycosyl diglyceride, digalactosyl diglyceride, galactosyl diglyceride and glycosyl diglyceride.

In an exemplary embodiment of the present disclosure, the sphingoglycolipid may be galactosyl cerebroside, lactosyl cerebroside or ganglioside.

In an exemplary embodiment of the present disclosure, the cationic lipid may be selected from a group consisting of 1,2-dioleoyl-3-trimethylammonium propane (DOTAP), N-(2,3-dioleyloxypropan-1-yl)-N,N,N-trimethylammonium chloride (DOTMA), 2,3-dioleyloxy-N-[2-(sperminecarboxamido)ethyl]-N,N-dimethyl-1-propanaminium trifluoroacetate (DOSPA), 1,2-dimyristyloxypropyl-3-dimethylhydroxyethylammonium bromide (DMRIE), 1,2-dioleoyloxypropyl-3-diethylhydroxyethylammonium bromide (DORIE) and 3β-[N-(N'N'-dimethylaminoethylhy)carbamoyl]cholesterol (DC-Chol).

In an exemplary embodiment of the present disclosure, the blood coagulation factor VIII may be composed of an amino acid sequence represented by SEQ ID NO 1, and the variant of blood coagulation factor VIII may be composed of an amino acid sequence represented by SEQ ID NO 3.

In an exemplary embodiment of the present disclosure, the composition may increase the expression of the blood coagulation factor gene by 30% or more.

The present disclosure also provides a pharmaceutical composition for preventing or treating a bleeding disorder or bleeding, which contains the composition described above and a human blood coagulation factor VIII gene or a variant gene thereof.

In an exemplary embodiment of the present disclosure, the bleeding disorder may be hemophilia A, hemophilia induced or complicated by an inhibitory antibody against blood coagulation factor VIII or blood coagulation factor Villa, or hemophilia B.

In an exemplary embodiment of the present disclosure, the bleeding disorder or bleeding may be one selected from a group consisting of neonatal coagulopathy, severe liver disease, thrombocytopenia, congenital deficiency of factor V, VII, X or XI, and von Willebrand disease having an inhibitor against von Willebrand factor.

In an exemplary embodiment of the present disclosure, the bleeding may be caused by blood loss associated with high-risk surgical procedures, traumatic blood loss, blood loss caused by bone marrow transplantation or blood loss caused by cerebral hemorrhage.

### [Advantageous Effects]

A composition for increasing the expression of a blood coagulation factor gene of the present disclosure may increase the expression of the blood coagulation factor gene by at least 30% when administered *in vivo* along with blood coagulation factor VIII gene or a variant gene thereof (e.g., a polynucleotide encoding the gene or a vector including the same). Accordingly, it is useful since a therapeutic effect can be achieved with a very small amount of the gene when the composition is administered along with a gene therapeutic agent.

### [Brief Description of Drawings]

FIG. 1 schematically illustrates a process of designing a blood coagulation factor VIII variant (F8M) according to an exemplary embodiment of the present disclosure.
FIG. 2 shows the cleavage map of a pGP vector according to an exemplary embodiment of the present disclosure.
FIG. 3 compares the expression level of the F8 protein in mouse depending on the administration of pGP-F8M (gene only), a pharmaceutical composition of a control group (F8M-JetPEI) and pharmaceutical compositions according to Example (F8M-MP1 and F8M-MP2).
FIG. 4 compares the expression level of the F9 protein in mouse depending on the administration of pGP-F9 (gene only) and pharmaceutical compositions according to Comparative Example (F9-MP1 and F9-MP2).
FIG. 5 schematically shows the domain structure constituting human blood coagulation factor VIII according to an exemplary embodiment of the present disclosure.

### [Best Mode]

Hereinafter, the present disclosure is described in detail.

In an aspect, the present disclosure provides a composition for increasing the expression of a blood coagulation factor gene, which contains core-shell structured microparticles as an active ingredient, wherein the core is a halogenated hydrocarbon, halogenated sulfur or a mixture thereof as a biocompatible gas, and the shell is a lipid or a derivative thereof, and the blood coagulation factor gene is one or more gene selected from a human blood coagulation factor VIII gene or a variant gene thereof.

### Core

In the present disclosure, the "core" may be composed of a halogenated hydrocarbon, halogenated sulfur or a mixture thereof as a biocompatible gas.

The biocompatible gas may be sulfur hexafluoride, octafluoropropane, bromochlorodifluoromethane, chlorodifluoromethane, dichlorodifluoromethane, bromotrifluoromethane, chlorotrifluoromethane, chloropentafluoroethane, dichlorotetrafluoroethane or a mixture thereof.

Specifically, the halogenated hydrocarbon may be a perfluorinated hydrocarbon.

The perfluorinated hydrocarbon may be perfluoromethane, perfluoroethane, perfluoropropane, perfluorobutane, perfluoropentane, perfluorohexane, perfluoroheptane, perfluoropropene, perfluorobutene, perfluorobutadiene, perfluorobut-2-ene, perfluorocyclobutane, perfluoromethylcyclobutane, perfluorodimethylcyclobutane, perfluorotrimethylcyclobutane, perfluorocyclopentane, perfluoromethylcyclopentane, perfluorodimethylcyclopentane, perfluoromethylcyclohexane, perfluoromethylcyclohexane, perfluoromethylcyclohexane or a mixture thereof.

Specifically, the biocompatible gas of the present disclosure may be sulfur hexafluoride or perfluorobutane.

### Shell

In the present disclosure, the "shell" may be composed of a lipid or a derivative thereof.

The lipid may be one or more selected from a group consisting of a simple lipid, a phospholipid, a glyceroglycolipid, a sphingoglycolipid, a cholesterol and a cationic lipid. Specifically, it may be a phospholipid.

The phospholipid may be a phosphatidylcholine derivative, a phosphatidylethanolamine derivative, a phosphatidylserine derivative, diacetylated phospholipid, L-α-dioleyl phosphatidylethanolamine, diolein, phosphatidic acid, phosphatidylglycerol, phosphatidylinositol, lysophosphatidylcholine, sphingomyelin, polyethylene glycolated phospholipid, egg yolk lecithin, soy lecithin, a hydrogenated phospholipid, etc.

The glyceroglycolipid may be sulfoxyribosyl glyceride, diglycosyl diglyceride, digalactosyl diglyceride, galactosyl diglyceride, glycosyl diglyceride, etc.

The sphingoglycolipid may be galactosyl cerebroside, lactosyl cerebroside, ganglioside, etc.

And, the cationic lipid may be 1,2-dioleoyl-3-trimethylammonium propane (DOTAP), N-(2,3-dioleyloxypropan-1-yl)-N,N,N-trimethylammonium chloride (DOTMA), 2,3-dioleyloxy-N-[2-(sperminecarboxamido)ethyl]-N,N-dimethyl-1-propanaminium trifluoroacetate (DOSPA), 1,2-dimyristyloxypropyl-3-dimethylhydroxyethylammonium bromide (DMRIE), 1,2-dioleoyloxypropyl-3-diethylhydroxyethylammonium bromide (DORIE), 3β-[N-(N'N'-dimethylaminoethylhy)carbamoyl]cholesterol (DC-Chol), etc.

### Microparticles

The microparticles of the present disclosure are stabilized by the shell which surrounds the core gas. The shell retards the diffusion of a gas to nearby liquid and prevents fusion between the microparticles.

When administered *in vivo,* the microparticle retains its shape until it reaches a target cell or tissue, and releases the gas as it is destroyed near the target cell or tissue. The released gas may cause change in the cell membrane of the target cell and may facilitate the entry of the growth factor gene into the cytoplasmic environment of the target cell via the jet force of the gas.

The microparticles may have an average diameter of 1-10 µm, specifically 2-8 µm, more specifically 2-4 µm.

### Composition for increasing gene expression

Recently, there have been many researches on core-shell microparticles having a gas as a core. In the previous researches, the effect of increasing gene expression was not achieved unless ultrasound was irradiated together with the microparticles.

Specifically, Sang-Chol Lee et al., Korean Circulation J 2006; 36: 32-38; "Enhancement of Gene Delivery into Mouse Skeletal Muscle with Microbubble Destruction by Low-Frequency Ultrasound" discloses that the effect of increasing gene expression is not achieved when only a luciferase gene-microparticles mixture is injected without irradiation of ultrasound.

ZP Shen et al., Gene Therapy (2008) 15, 1147-1155; "Ultrasound with microbubbles enhances gene expression of plasmid DNA in the liver via intraportal delivery" also discloses that the effect of increasing gene expression is not achieved when only a luciferase gene-microparticles mixture is injected without irradiation of ultrasound.

In addition, Xingsheng Li et al., J Ultrasound Med 2008; 27: 453-460; "Experimental Research on Therapeutic Angiogenesis Induced by Hepatocyte Growth Factor Directed by Ultrasound-Targeted Microbubble Destruction in Rats" discloses that the effect of increasing gene expression is not achieved when only a HGF gene-liposome microparticles mixture is injected without irradiation of ultrasound.

However, the inventors of the present disclosure have identified that, while studying on the use of microparticles, the expression level of a blood coagulation factor gene, particularly a blood coagulation factor VIII gene, is increased remarkably even without ultrasound irradiation when the gene is injected along with the microparticles according to the present disclosure, and have completed the present disclosure. Meanwhile, as described specifically in the test example described later, the effect of increasing gene expression was not achieved with the microparticles for blood coagulation factor genes other than the blood coagulation factor VIII gene or a variant gene thereof (e.g., blood coagulation factor IX (also known as factor IX, factor 9, FIX or F9)).

When administered *in vivo* along with a blood coagulation factor VIII gene or a variant gene thereof, the composition for increasing gene expression of the present disclosure can increase the expression level of the blood coagulation factor gene by at least 30%.

In the examples described below, it was verified that, when administered along with a human blood coagulation factor VIII variant gene into mouse, the composition for increasing gene expression of the present disclosure increases the expression level of the blood coagulation factor VIII gene remarkably. More specifically, it was verified that, when the composition for increasing gene expression of the present disclosure and the blood coagulation factor VIII variant gene are administered together into an animal model, the expression level of the blood coagulation factor VIII gene is increased by 40% or more. In contrast, for a blood coagulation factor IX gene, the expression of the gene was hardly increased even when the composition for increasing gene expression of the present disclosure was administered together into mouse.

That is to say, it seems that the composition for increasing gene expression of the present disclosure is effective in increasing the expression level of the human blood coagulation factor gene specifically only when administered along with one or more gene selected from human blood coagulation factor VIII gene and a variant gene thereof, from among blood coagulation factors.

The composition may further contain a pharmaceutical adjuvant such as a stabilizer, a buffer, a salt for control of osmotic pressure, an excipient, an antiseptic, etc. or other therapeutically useful substances, and may be prepared into various formulations for oral or parenteral administration, specifically for parenteral administration, according to common methods. Specifically, a formulation for parenteral administration may be typically an injection formulation in the form of an isotonic aqueous solution or suspension. Alternatively, the composition may be prepared into a powder and then suspended in a solvent immediately before administration.

In the composition for increasing gene expression of the present disclosure, the content of the microparticles may be 0.5-2,000 µL/mL, specifically 1-1,000 µL/mL or 5-2,000 µg/mL, specifically 10-1,000 µg/mL, although not being particularly limited.

If the content of the microparticles is outside the above range, the desired effect cannot be achieved.

Specifically, the composition may be administered as a mixture with a gene to achieve a better effect.

### Gene

The composition for increasing gene expression according to the present disclosure may further increase the expression and efficiency of genes when administered along with the following genes.

### Blood coagulation factor VIII

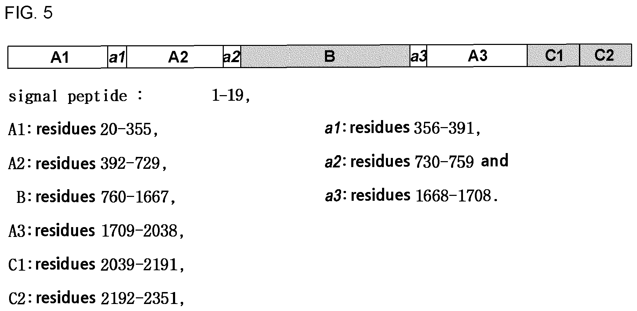

In the present disclosure, the terms "blood coagulation factor VIII", "factor VIII", "FVIII" and "F8" are used interchangeably. Mature human blood coagulation factor VIII is composed of 2351 amino acids (including signal peptides), which are arranged in a domain structure shown in FIG. 5.

In FIG. 5, a1, a2 and a3 are acidic domains. The acidic domain a3 is known to be involved in the binding of the blood coagulation factor VIII molecule to a von Willebrand factor (vWF), which plays an important role in blood coagulation. In the process of secretion, the blood coagulation factor VIII is cleaved between the B-domain and the a3 acidic domain, resulting in a heterodimeric polypeptide. The blood coagulation factor VIII heterodimer consists of a light chain (including A3, C1 and C2) and a heavy chain of variable size (including A1, A2 and B). The heavy chain is heterogeneous due to limited proteolysis in the B-domain. In case of heterodimeric B-domain-deleted blood coagulation factor VIII, the "heavy chain" includes A1 and A2 but the B-domain is deleted partially or wholly.

The amino acid sequence of mature wild-type human blood coagulation factor VIII is shown in SEQ ID NO 1. The reference of an amino acid position of a specific sequence means the position of the amino acid in the VIII wild-type protein and does not exclude the presence of mutations (e.g., deletion, insertion and/or substitution) at other positions in the sequence. A DNA sequence encoding SEQ ID NO 1 is represented by SEQ ID NO 2.

"Blood coagulation factor VIII" includes not only wild-type blood coagulation factor VIII but also derivatives of the wild-type blood coagulation factor VIII having the procoagulant activity of the wild-type blood coagulation factor VIII. The derivative may have a sequence with deletion, insertion and/or addition as compared to the amino acid sequence of the wild-type blood coagulation factor VIII. Specifically, the derivative may be a molecule of blood coagulation factor VIII with all or part of the B-domain deleted. Throughout the present disclosure, the positions amino acids always refer to the positions of individual amino acids in the full-length mature wild-type blood coagulation factor VIII (including signal peptides).

### Blood coagulation factor VIII variant

In the present disclosure, the term "variant" includes conservative or non-conservative substitution, insertion or deletion of an amino acid sequence, a nucleic acid sequence, etc., and such change does not substantially alter the active site or active domain that confers the biological activity of FVIII.

The blood coagulation factor VIII variant according to the present disclosure is a single-chain blood coagulation factor VIII variant in which amino acids Asp784 to Arg1671 are deleted from blood coagulation factor VIII represented by SEQ ID NO 1. The blood coagulation factor VIII variant is one in which a part of the B-domain (residues 784-1667) and a part of the a3 domain (residues 1668-1671) are deleted, and the variant exhibits enhanced protein expression and improved blood coagulation activity and stability as compared to blood coagulation factor VIII and B-domain-deleted blood coagulation factor VIII. The blood coagulation factor VIII variant has an amino acid sequence represented by SEQ ID NO 3.

The "single-chain blood coagulation factor VIII" means a blood coagulation factor VIII molecule which exists as a single polypeptide chain without being cleaved into tow chains (e.g., a heavy chain and a light chain) by proteolysis during secretion from a cell expressing the blood coagulation factor VIII molecule.

In addition, the blood coagulation factor VIII variant may be expressed as a polynucleotide encoding the amino acid sequence.

### Polynucleotide

In the present disclosure, the term "polynucleotide(s)" refers to any polyribonucleotide or polydeoxyribonucleotide which may be an unmodified RNA or DNA or a modified RNA or DNA. The polynucleotide of the present invention may be a single-chain DNA or RNA. As used herein, the term "polynucleotide(s)" includes a DNA or RNA containing modified bases and/or unique bases such as inosine. It is obvious that various modifications can be made to the DNA or RNA to serve a known useful purpose. As used herein, the term "polynucleotide(s)" includes such chemically, enzymatically or metabolically modified polynucleotide(s).

Those skilled in the art will appreciate that the blood coagulation factor VIII variant may be encoded by several polynucleotides due to the degeneracy of the genetic code. In other words, a polynucleotide sequence encoding the blood coagulation factor VIII variant that can be used in the present invention is interpreted to include a nucleotide sequence showing substantial identity to the amino acid sequence.

### Plasmid

When the composition for increasing gene expression according to the present disclosure is administered along with a plasmid containing a single-chain polynucleotide encoding the gene, the expression efficiency and efficacy of the gene may be increased further.

In the present disclosure, the term "plasmid" generally refers to a circular DNA molecule formed by being operably linked to a vector so that an exogenous gene can be expressed in a host cell. However, the plasmid can be used as a vector that is degraded by specific restriction enzymes by gene recombination to incorporate a desired gene. Thus, the terms plasmid and vector are used interchangeably herein, and those skilled in the art of genetic engineering will fully understand their meanings even if they are not distinguished by names.

In the present disclosure, the term "vector" refers to a DNA molecule as a carrier that can stably transport an exogenous gene into a host cell. To be a useful vector, it must be replicable, have a way to enter the host cell, and be equipped with a means to detect its presence.

### Expression

### Expression vector

The composition for increasing gene expression according to the present disclosure can further increase the expression and efficiency of a gene when administered along with expression vectors including single-chain polynucleotides encoding the gene.

In the present disclosure, the term "expression" refers to generation of the gene in a cell.

In the present disclosure, the term "expression vector" refers to a vector capable of expressing a target gene in a suitable host, and means a gene construct including an essential regulatory element operably linked to express a gene insert.

In the present disclosure, the term "operably linked" means that a nucleic acid expression-regulating sequence and a polynucleotide encoding a target gene are functionally linked to perform a general function. For example, a promoter and a polynucleotide encoding the gene may be operably linked to affect the expression of the polynucleotide. The operable linkage to a recombinant vector may be prepared using a genetic recombinant technique well known in the art, and site-specific DNA cleavage and ligation may be achieved using enzymes generally known in the art.

The expression vector of the present disclosure may be prepared using a plasmid, a vector or a viral vector, although not being limited thereto. An appropriate expression vector may include an expression-regulating element such as a promoter, an operator, a start codon, a stop codon, a polyadenylation signal, an enhancer, etc. and may be prepared variously according to purposes. The promoter of the vector may be constitutive or inducible. Since a plasmid is the most commonly used form of a vector at present, the terms "plasmid" and "vector" are used sometimes interchangeably in the present disclosure. For the purpose of the present disclosure, it is preferred to use a plasmid vector. A typical plasmid vector that can be used for this purpose has a structure including (a) a replication origin for effective replication into several to hundreds of plasmid vectors per host cell and (b) a restriction enzyme site into which a fragment of foreign DNA can be inserted. Even if a proper restriction enzyme site does not exist, the vector and foreign DNA can be easily ligated using synthetic oligonucleotide adaptors or linkers according to common methods.

A vector used for overexpression of a gene according to the present disclosure may be an expression vector known in the art. A framework vector that may be used in the present disclosure may be selected from a group consisting of pCDNA3.1, pGP, pEF, pVAX, pUDK, pCK, pQE40, pT7, pET/Rb, pET28a, pET-22b(+) and pGEX, although not being particularly limited thereto. Specifically, use of a vector selected from a group consisting of pGP, pEF, pCK, pUDK and pVAX may be preferred in terms of effect.

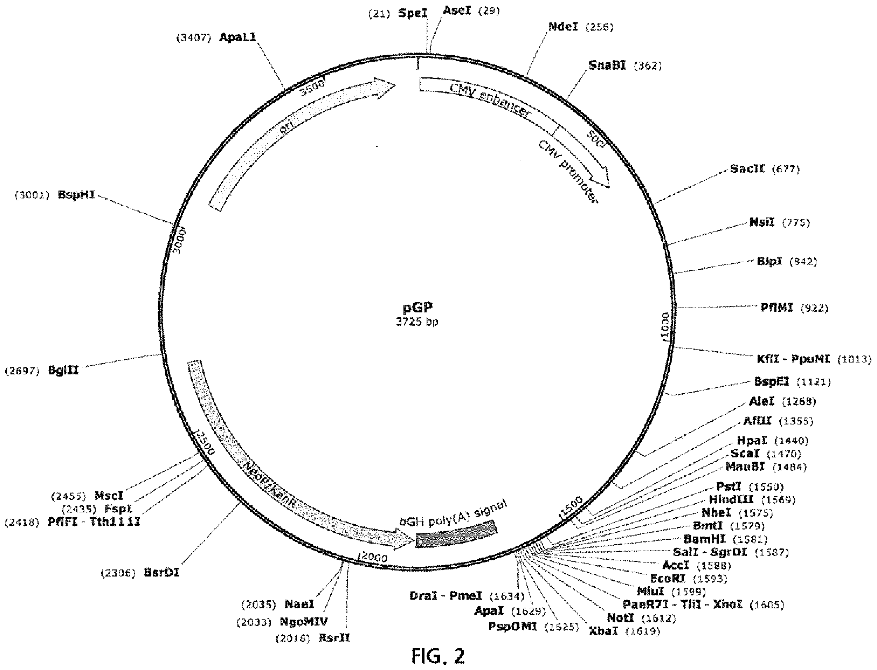

In a specific exemplary embodiment, the expression vector of the present disclosure may be an expression vector including a pGP vector having a cleavage map of FIG. 2.

### Pharmaceutical composition

In another aspect, the present disclosure provides a pharmaceutical composition for preventing or treating a bleeding disorder or bleeding, which contains the composition for increasing gene expression of the present disclosure and the blood coagulation factor VIII gene or a variant gene thereof.

The bleeding disorder may be hemophilia A, hemophilia induced or complicated by an inhibitory antibody against blood coagulation factor VIII or blood coagulation factor Villa, or hemophilia B. In addition, the bleeding disorder or bleeding may be one selected from a group consisting of neonatal coagulopathy, severe liver disease, thrombocytopenia, congenital deficiency of factor V, VII, X or XI, and von Willebrand disease having an inhibitor against von Willebrand factor.

And, the bleeding may be one caused by blood loss associated with high-risk surgical procedures, traumatic blood loss, blood loss caused by bone marrow transplantation or blood loss caused by cerebral hemorrhage.

The pharmaceutical composition for preventing or treating a bleeding disorder or bleeding may be usefully used to prevent or treat a bleeding disorder or bleeding since it exhibits superior therapeutic effect because gene expression is increased *in vivo* even with a small amount of the blood coagulation factor VIII gene or a variant gene thereof.

In the pharmaceutical composition of the present disclosure, the composition for increasing gene expression and the blood coagulation factor VIII gene or a variant gene thereof may be contained with a volume ratio of 1 : 0.5-30 (w/v).

The pharmaceutical composition of the present disclosure may be for gene therapy.

### Formulation

The pharmaceutical composition of the present disclosure may be prepared into pharmaceutical formulations for therapeutic purposes.

Pharmaceutical carriers and excipients and suitable pharmaceutical formulations are known in the art (e.g., "Pharmaceutical Formulation Development of Peptides and Proteins", Frokjaer et al., Taylor & Francis (2000) or "Handbook of Pharmaceutical Excipients", 3rd edition, Kibbe et al., Pharmaceutical Press (2000)). In particular, the pharmaceutical composition of the present disclosure may be formulated as a lyophilized form or a stable liquid form. The composition of the present disclosure may be freeze-dried through various procedures known in the art. The freeze-dried formulation is reconstituted by adding one or more pharmaceutically acceptable diluent such as sterile water for injection or sterile physiological saline.

The formulation of the composition is delivered to a subject via any pharmaceutical suitable means of administration. Various known delivery systems may be used to deliver the composition through any convenient routes. Mainly, the composition of the present disclosure is administered systemically. For systemic administration, the composition of the present disclosure is formulated for parenteral (e.g., intravenous, subcutaneous, intramuscular, intraperitoneal, intracerebral, intrapulmonary, intranasal or transdermal) delivery or enteral (e.g., oral, vaginal or rectal) delivery according to common methods. The most preferred routes of administration are intravenous and intramuscular routes. These formulations can be administered continuously by infusion or by bolus injection. Some formulations encompass slow release systems.

The composition of the present disclosure is administered to a patient in a therapeutically effective dose, meaning a dose that is sufficient to produce the desired effect, preventing or lessening the severity or spread of the condition or indication being treated without reaching a dose which produces intolerable adverse effects. The exact dose depends on many factors such as the indication, formulation, mode of administration, etc. and has to be determined through preclinical and clinical trials for each respective indication.

The pharmaceutical composition of the present disclosure may be administered either alone or in combination with other therapeutic agents. These agents may be incorporated as a part of the same pharmaceutical.

### Treatment method

The present disclosure also relates to a method for treating a subject suffering from a bleeding disorder such as hemophilia A, hemophilia B or acquired hemophilia or a subject suffering from a chronic disease or liver disease. The treatment method may include a step of administering an effective amount of the pharmaceutical composition of the present disclosure to the subject.

According to an exemplary embodiment of the present disclosure, the gene of the present disclosure such as blood coagulation factor VIII gene or a variant gene thereof may be administered at a dose of 10 ng to 100 mg. When the administration of the blood coagulation factor VIII, a variant thereof or a polynucleotide encoding the same is repeated more than once, the administration dose may be the same or different for each administration.

Hereinafter, the present disclosure is described in more detail through specific examples. However, the examples are only for illustrating the present disclosure in more detail and it will be obvious to those of ordinary skill in the art that the scope of the present disclosure is not limited by them.

### [Examples]

### Preparation of materials

### Genes

### Blood coagulation factor VIII (F8)

A gene full-length blood coagulation factor VIII (F8) represented by SEQ ID 1 was synthesized by Genscript (USA).

### Variant of blood coagulation factor VIII (F8M)

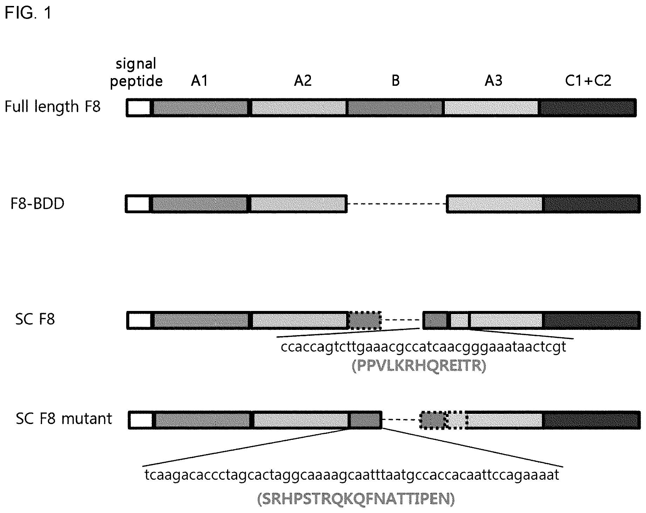

A full-length blood coagulation factor VIII gene represented by SEQ ID NO 1 was used as a template, and fragment 1 represented by SEQ ID NO 4 was prepared through polymerase chain reaction (PCR) using primers 1 and 2. The PCR was performed by mixing 2 µL of the template DNA, 1 µL of 10 µmol/µL primer each, 2.5 µL of 2.5 mM dNTP, 1 µL of a pfu enzyme mix (Enzynomics, Korea), 2.5 µL of a 10x buffer, and 50 µL of sterilized triply distilled water. The solution was reacted for 30 seconds at 95 °C, 30 seconds at 60 °C and 30 seconds at 72 °C for 40 cycles. Using primers 3 and 4, fragment 2 represented by SEQ ID NO 5 was prepared by PCR as in the same manner as described above. Subsequently, the fragments 1 and 2 were subjected to overlapping PCR using primers 5 and 6, thereby preparing a single-chain blood coagulation factor VIII variant. In the overlapping PCR, 2 µL of each DNA fragment was added, and reaction time and method were the same as described above in the PCR. FIG. 1 schematically illustrates the process of designing the blood coagulation factor VIII variant (F8M) according to an exemplary embodiment of the present disclosure.

The primers are summarized in Table 1 below.

**[Table 1]**

| Primer No. | Primer name | Base sequence |
|---|---|---|
| 1 | F8(F) | ATGCAAATAGAGCTCTCCACCTGCTTCTTT |
| 2 | F8M-1 (R) | TCTGACTGAAGAGTAGTATTTTCTGGAATTGTGGT GGCATTAAAT |
| 3 | F8M-2(F) | CCACAATTCCAGAAAATACTACTCTTCAGTCAGAT CAAGAGGAAATTGAC |
| 4 | F8(R) | TCAGTAGAGGTCCTGTGCCTCGCAGCCCAG |
| 5 | F8 final(F) | GCTAGCATGCAAATAGAGCTCTCCACCTGC |
| 6 | F8 final(R) | GCGGCCGCTCAGTAGAGGTCCTGTGCCTCGCA |

### Blood coagulation factor IX (F9)

A gene of full-length blood coagulation factor IX (see GenBank base sequence FR846239.1) represented by SEQ ID NO 6 was synthesized by Bionics (Korea).

### Plasmids (pGP)

After synthesizing pCK plasmids referring to the literature Lee *et al.* (Lee Y, et al. Improved expression of vascular endothelial growth factor by naked DNA in mouse skeletal muscles: implication for gene therapy of ischemic diseases. Biochem. Biophys. Res. Commun. 2000; 272(1): 230-235), PCR was conducted in the same manner described in the literature using primers 7 and 8 of Table 2 below. After reacting the obtained fragments with EcoRI enzyme at 37 °C for 1 hour, DNA was purified using an Expin Gel SV (GeneAII, Korea) kit. Then, after conducting ligation for 30 minutes using T4 ligase, the DNA was incubated overnight with *E*. *coli.* Next day, after isolating DNA from the colony through mini-prep, pGP plasmids represented by SEQ ID NO 7 were obtained. FIG. 2 shows the cleavage map of the pGP vector according to an exemplary embodiment of the present disclosure.

**[Table 2]**

| Primer No. | Primer name | Base sequence |
|---|---|---|
| 7 | pGP(F) | GACGAATTCACGCGTCTCGAGGCGGCCGCTCTA GAGGGCCCGTTTAAA |
| 8 | pGP(R) | GACGAATTCGTCGACGGATCCGCTAGCAAGCTT CGTGTCAAGGACGGT |

### Preparation Example

### Preparation of plasmid DNAs including genes

Each of the genes and each of the pGP plasmids prepared above were cleaved with Nhel and Notl enzymes for 1 hour and fragments were separated by conducting electrophoresis on agarose gel. The separated fragments were ligated for 30 minutes using T4 ligase and then incubated overnight with *E*. *coli.* Next day, DNA was isolated from the colony through mini-prep, and then digested with Nhel and Notl. The cloned DNA was incubated overnight with an *E*. *coli* supernatant digested with restriction enzymes in a 4-L flask in the presence of kanamycin. Plasmid DNAs produced using an Endofree Giga prep. kit (Qiagen, USA) were used in animal experiments. The prepared plasmid DNAs are summarized in Table 3.

**[Table 3]**

| Gene | Plasmid DNA |
|---|---|
| Blood coagulation factor VIII (F8) | pGP-F8 |
| Blood coagulation factor VIII variant (F8M) | pGP-F8M |
| Blood coagulation factor IX (F9) | pGP-F9 |

### Examples

### Example: Preparation of pharmaceutical composition containing composition for increasing expression of gene and F8M (F8M-MP1 and F8M-MP2)

### Composition for increasing gene expression

Core-shell structured microparticles with a reference code of 621400210, having an average diameter of about 2.5 µm and composed of sulfur hexafluoride as a core and a lipid as a shell, were purchased from Bracco Imaging Korea (MP1). In addition, core-shell structured microparticles with a reference code of 646300210, having an average diameter of about 2.4-3.6 µm and composed of perfluorobutane as a core and a shell including a lipid and a surfactant, were purchased from GE Healthcare Korea (MP2).

### Preparation of pharmaceutical composition containing composition for increasing gene expression and F8M

Pharmaceutical compositions F8M-MP1 and F8M-MP2 were prepared by mixing 15 µL of the compositions for increasing gene expression (MP1 and MP2), respectively, with the pGP-F8M prepared above (70 µg/35 µL).

### Comparative Example: Preparation of pharmaceutical composition containing composition for increasing gene expression and F9 (F9-MP1 and F9-MP2)

### Composition for increasing gene expression

Compositions for increasing gene expression were prepared in the same manner as in Example.

### Preparation of pharmaceutical composition containing composition for increasing gene expression and F9

Pharmaceutical compositions F9-MP1 and F9-MP2 were prepared by mixing 15 µL of the compositions for increasing gene expression (MP1 and MP2), respectively, with the pGP-F9 prepared above (70 µg/35 µL).

### Control group

### Composition for increasing gene expression

A suspension (corresponding to a composition for increasing gene expression) was prepared by mixing 128 µL of *in vivo* JetPEI (Polyplus, USA) with 2 mL of 5% glucose according to the manufacturer's manual.

### Preparation of pharmaceutical composition containing composition for increasing gene expression and each gene

A pharmaceutical compositions F8M-JetPEI was prepared by mixing 15 µL of the composition with the pGP-F8M prepared above (70 µg/35 µL).

### Test Example

### Test Example: Expression level of protein in mouse

Each of the pharmaceutical compositions according to the control group, example and comparative example was injected into the lower calf muscle of C57bl/6 mouse with 75 µg/50 µL/leg.

On day 7 after the administration, the mouse was sacrificed and the muscle at the injected area was excised. Then, total proteins were isolated after grinding the excised muscle was using liquid nitrogen and a protein extraction kit (Cell Biolabs, USA). The amounts of the isolated total proteins were measured using a DC protein assay kit (Bio-Rad laboratories, USA).

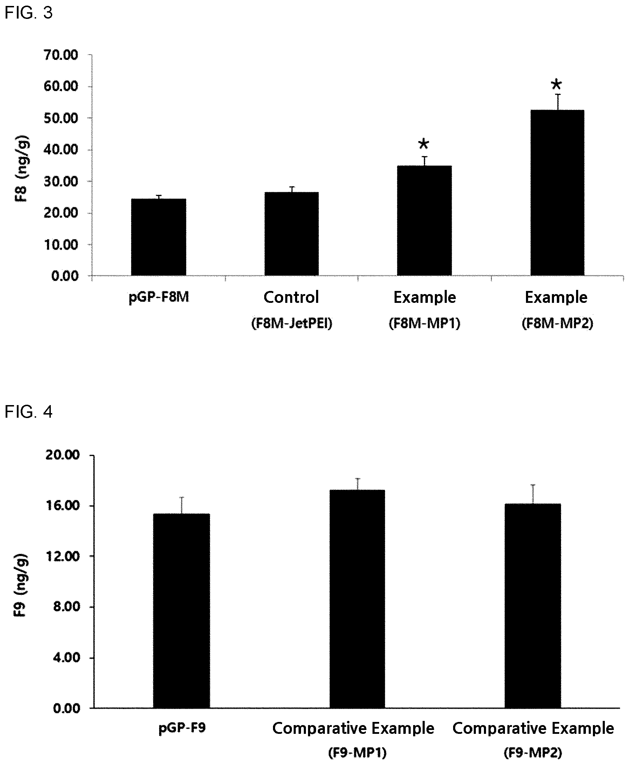

For measurement of the F8 protein, the expression level of each gene was measured using an ELISA kit (Stago Asserchrom VIII:Ag, France) for the same amount of protein. The result is shown in FIG. 3.

From FIG. 3, it can be seen that the administration of the pharmaceutical composition according to the present disclosure (Example) resulted in a statistically significantly higher expression level as compared to the administration of the gene only or the administration of the composition of the control group. Specifically, the group administered with the F8M-MP1 of Example showed 44% higher expression level as compared to group administered with the gene only (pGP-F8M), and the group administered with F8M-MP2 showed 116% higher expression level as compared to group administered with the gene only.

For measurement of the F9 protein, the expression level of each gene was measured using an ELISA kit (Abcam, USA) for the same amount of protein. The result is shown in FIG. 4.

From FIG. 4, it can be seen that the administration of the composition of Comparative Example hardly resulted in increased gene expression as compared to the administration of the gene only.

That is to say, from FIG. 3 and FIG. 4, it can be seen that the administration of the compositions according to the present disclosure (Example) results in a significantly higher expression level as compared to the administration of the gene only or the administration of the composition of the control group or comparative example.

Although the present disclosure was illustrated with the specific exemplary embodiments described above, various modifications or changes can be made thereto without departing from the subject matter and scope of the present disclosure. In addition, such modifications or changes within the subject matter of the present disclosure are included in the scope of the appended claims.

## Claims

1. A composition for increasing the expression of a blood coagulation factor gene, comprising core-shell structured microparticles as an active ingredient, wherein
the core is a halogenated hydrocarbon, halogenated sulfur or a mixture thereof as a biocompatible gas, and the shell is a lipid or a derivative thereof, and
the blood coagulation factor gene is one or more gene selected from a human blood coagulation factor VIII gene or a variant gene thereof.

2. The composition for increasing the expression of a blood coagulation factor gene according to claim 1, wherein the biocompatible gas is selected from sulfur hexafluoride, octafluoropropane, bromochlorodifluoromethane, chlorodifluoromethane, dichlorodifluoromethane, bromotrifluoromethane, chlorotrifluoromethane, chloropentafluoroethane, dichlorotetrafluoroethane and a mixture thereof.

3. The composition for increasing the expression of a blood coagulation factor gene according to claim 1, wherein the halogenated hydrocarbon is a perfluorinated hydrocarbon.

4. The composition for increasing the expression of a blood coagulation factor gene according to claim 3, wherein the perfluorinated hydrocarbon is perfluoromethane, perfluoroethane, perfluoropropane, perfluorobutane, perfluoropentane, perfluorohexane, perfluoroheptane, perfluoropropene, perfluorobutene, perfluorobutadiene, perfluorobut-2-ene, perfluorocyclobutane, perfluoromethylcyclobutane, perfluorodimethylcyclobutane, perfluorotrimethylcyclobutane, perfluorocyclopentane, perfluoromethylcyclopentane, perfluorodimethylcyclopentane, perfluoromethylcyclohexane, perfluoromethylcyclohexane, perfluoromethylcyclohexane or a mixture thereof.

5. The composition for increasing the expression of a blood coagulation factor gene according to claim 1, wherein the lipid is one or more selected from a group consisting of a simple lipid, a phospholipid, a glyceroglycolipid, a sphingoglycolipid, a cholesterol and a cationic lipid.

6. The composition for increasing the expression of a blood coagulation factor gene according to claim 5, wherein the phospholipid is selected from a group consisting of a phosphatidylcholine derivative, a phosphatidylethanolamine derivative, a phosphatidylserine derivative, a diacetylated phospholipid, L-α-dioleyl phosphatidylethanolamine, diolein, phosphatidic acid, phosphatidylglycerol, phosphatidylinositol, lysophosphatidylcholine, sphingomyelin, a polyethylene glycolated phospholipid, egg yolk lecithin, soy lecithin and a hydrogenated phospholipid.

7. The composition for increasing the expression of a blood coagulation factor gene according to claim 5, wherein the glyceroglycolipid is selected from a group consisting of sulfoxyribosyl glyceride, diglycosyl diglyceride, digalactosyl diglyceride, galactosyl diglyceride and glycosyl diglyceride.

8. The composition for increasing the expression of a blood coagulation factor gene according to claim 5, wherein the sphingoglycolipid is galactosyl cerebroside, lactosyl cerebroside or ganglioside.

9. The composition for increasing the expression of a blood coagulation factor gene according to claim 5, wherein the cationic lipid is selected from a group consisting of 1,2-dioleoyl-3-trimethylammonium propane (DOTAP), N-(2,3-dioleyloxypropan-1-yl)-N,N,N-trimethylammonium chloride (DOTMA), 2,3-dioleyloxy-N-[2-(sperminecarboxamido)ethyl]-N,N-dimethyl-1-propanaminium trifluoroacetate (DOSPA), 1,2-dimyristyloxypropyl-3-dimethylhydroxyethylammonium bromide (DMRIE), 1,2-dioleoyloxypropyl-3-diethylhydroxyethylammonium bromide (DORIE) and 3β-[N-(N'N'-dimethylaminoethylhy)carbamoyl]cholesterol (DC-Chol).

10. The composition for increasing the expression of a blood coagulation factor gene according to claim 1, wherein the blood coagulation factor VIII is composed of an amino acid sequence represented by SEQ ID NO 1, and the variant of blood coagulation factor VIII is composed of an amino acid sequence represented by SEQ ID NO 3.

11. The composition for increasing the expression of a blood coagulation factor gene according to claim 1, wherein the composition increases the expression of the blood coagulation factor gene by 30% or more.

12. A pharmaceutical composition for preventing or treating a bleeding disorder or bleeding, comprising the composition according to claim 1 and a human blood coagulation factor VIII gene or a variant gene thereof.

13. The pharmaceutical composition for preventing or treating a bleeding disorder or bleeding according to claim 12, wherein the bleeding disorder is hemophilia A, hemophilia induced or complicated by an inhibitory antibody against blood coagulation factor VIII or blood coagulation factor Villa, or hemophilia B.

14. The pharmaceutical composition for preventing or treating a bleeding disorder or bleeding according to claim 12, wherein the bleeding disorder or bleeding is one of neonatal coagulopathy, severe liver disease, thrombocytopenia, congenital deficiency of factor V, VII, X or XI, and von Willebrand disease having an inhibitor against von Willebrand factor.

15. The pharmaceutical composition for preventing or treating a bleeding disorder or bleeding according to claim 12, wherein the bleeding is caused by blood loss associated with high-risk surgical procedures, traumatic blood loss, blood loss caused by bone marrow transplantation or blood loss caused by cerebral hemorrhage.


<110>    GNPBIOSCIENCE

<120>    Composition for increasing expression level of blood coagulation
         factor genes containing core-shell structured microparticles as
         effective component

<130>    HPC-8492

<150>    KR 2018/0017075
<151>    2018-02-12

<160>    7

<170>    KoPatentIn 3.0

<210>    1
<211>    2351
<212>    PRT
<213>    Artificial Sequence

<220>
<223>    amino acid sequence of Factor 8


<400>    1
Met Gln Ile Glu Leu Ser Thr Cys Phe Phe Leu Cys Leu Leu Arg Phe
  1               5                  10                  15

Cys Phe Ser Ala Thr Arg Arg Tyr Tyr Leu Gly Ala Val Glu Leu Ser
             20                  25                  30

Trp Asp Tyr Met Gln Ser Asp Leu Gly Glu Leu Pro Val Asp Ala Arg
         35                  40                  45

Phe Pro Pro Arg Val Pro Lys Ser Phe Pro Phe Asn Thr Ser Val Val
     50                  55                  60

Tyr Lys Lys Thr Leu Phe Val Glu Phe Thr Asp His Leu Phe Asn Ile
 65                  70                  75                  80

Ala Lys Pro Arg Pro Pro Trp Met Gly Leu Leu Gly Pro Thr Ile Gln
                 85                  90                  95

Ala Glu Val Tyr Asp Thr Val Val Ile Thr Leu Lys Asn Met Ala Ser
            100                 105                 110

His Pro Val Ser Leu His Ala Val Gly Val Ser Tyr Trp Lys Ala Ser
        115                 120                 125

Glu Gly Ala Glu Tyr Asp Asp Gln Thr Ser Gln Arg Glu Lys Glu Asp
    130                 135                 140

Asp Lys Val Phe Pro Gly Gly Ser His Thr Tyr Val Trp Gln Val Leu
145                 150                 155                 160

Lys Glu Asn Gly Pro Met Ala Ser Asp Pro Leu Cys Leu Thr Tyr Ser
                165                 170                 175

Tyr Leu Ser His Val Asp Leu Val Lys Asp Leu Asn Ser Gly Leu Ile
            180                 185                 190

Gly Ala Leu Leu Val Cys Arg Glu Gly Ser Leu Ala Lys Glu Lys Thr
        195                 200                 205

Gln Thr Leu His Lys Phe Ile Leu Leu Phe Ala Val Phe Asp Glu Gly
    210                 215                 220

Lys Ser Trp His Ser Glu Thr Lys Asn Ser Leu Met Gln Asp Arg Asp
225                 230                 235                 240

Ala Ala Ser Ala Arg Ala Trp Pro Lys Met His Thr Val Asn Gly Tyr
                245                 250                 255

Val Asn Arg Ser Leu Pro Gly Leu Ile Gly Cys His Arg Lys Ser Val
            260                 265                 270

Tyr Trp His Val Ile Gly Met Gly Thr Thr Pro Glu Val His Ser Ile
        275                 280                 285

Phe Leu Glu Gly His Thr Phe Leu Val Arg Asn His Arg Gln Ala Ser
    290                 295                 300

Leu Glu Ile Ser Pro Ile Thr Phe Leu Thr Ala Gln Thr Leu Leu Met
305                 310                 315                 320

Asp Leu Gly Gln Phe Leu Leu Phe Cys His Ile Ser Ser His Gln His
                325                 330                 335

Asp Gly Met Glu Ala Tyr Val Lys Val Asp Ser Cys Pro Glu Glu Pro
            340                 345                 350

Gln Leu Arg Met Lys Asn Asn Glu Glu Ala Glu Asp Tyr Asp Asp Asp
        355                 360                 365

Leu Thr Asp Ser Glu Met Asp Val Val Arg Phe Asp Asp Asp Asn Ser
    370                 375                 380

Pro Ser Phe Ile Gln Ile Arg Ser Val Ala Lys Lys His Pro Lys Thr
385                 390                 395                 400

Trp Val His Tyr Ile Ala Ala Glu Glu Glu Asp Trp Asp Tyr Ala Pro
                405                 410                 415

Leu Val Leu Ala Pro Asp Asp Arg Ser Tyr Lys Ser Gln Tyr Leu Asn
            420                 425                 430

Asn Gly Pro Gln Arg Ile Gly Arg Lys Tyr Lys Lys Val Arg Phe Met
        435                 440                 445

Ala Tyr Thr Asp Glu Thr Phe Lys Thr Arg Glu Ala Ile Gln His Glu
    450                 455                 460

Ser Gly Ile Leu Gly Pro Leu Leu Tyr Gly Glu Val Gly Asp Thr Leu
465                 470                 475                 480

Leu Ile Ile Phe Lys Asn Gln Ala Ser Arg Pro Tyr Asn Ile Tyr Pro
                485                 490                 495

His Gly Ile Thr Asp Val Arg Pro Leu Tyr Ser Arg Arg Leu Pro Lys
            500                 505                 510

Gly Val Lys His Leu Lys Asp Phe Pro Ile Leu Pro Gly Glu Ile Phe
        515                 520                 525

Lys Tyr Lys Trp Thr Val Thr Val Glu Asp Gly Pro Thr Lys Ser Asp
    530                 535                 540

Pro Arg Cys Leu Thr Arg Tyr Tyr Ser Ser Phe Val Asn Met Glu Arg
545                 550                 555                 560

Asp Leu Ala Ser Gly Leu Ile Gly Pro Leu Leu Ile Cys Tyr Lys Glu
                565                 570                 575

Ser Val Asp Gln Arg Gly Asn Gln Ile Met Ser Asp Lys Arg Asn Val
            580                 585                 590

Ile Leu Phe Ser Val Phe Asp Glu Asn Arg Ser Trp Tyr Leu Thr Glu
        595                 600                 605

Asn Ile Gln Arg Phe Leu Pro Asn Pro Ala Gly Val Gln Leu Glu Asp
    610                 615                 620

Pro Glu Phe Gln Ala Ser Asn Ile Met His Ser Ile Asn Gly Tyr Val
625                 630                 635                 640

Phe Asp Ser Leu Gln Leu Ser Val Cys Leu His Glu Val Ala Tyr Trp
                645                 650                 655

Tyr Ile Leu Ser Ile Gly Ala Gln Thr Asp Phe Leu Ser Val Phe Phe
            660                 665                 670

Ser Gly Tyr Thr Phe Lys His Lys Met Val Tyr Glu Asp Thr Leu Thr
        675                 680                 685

Leu Phe Pro Phe Ser Gly Glu Thr Val Phe Met Ser Met Glu Asn Pro
    690                 695                 700

Gly Leu Trp Ile Leu Gly Cys His Asn Ser Asp Phe Arg Asn Arg Gly
705                 710                 715                 720

Met Thr Ala Leu Leu Lys Val Ser Ser Cys Asp Lys Asn Thr Gly Asp
                725                 730                 735

Tyr Tyr Glu Asp Ser Tyr Glu Asp Ile Ser Ala Tyr Leu Leu Ser Lys
            740                 745                 750

Asn Asn Ala Ile Glu Pro Arg Ser Phe Ser Gln Asn Ser Arg His Pro
        755                 760                 765

Ser Thr Arg Gln Lys Gln Phe Asn Ala Thr Thr Ile Pro Glu Asn Asp
    770                 775                 780

Ile Glu Lys Thr Asp Pro Trp Phe Ala His Arg Thr Pro Met Pro Lys
785                 790                 795                 800

Ile Gln Asn Val Ser Ser Ser Asp Leu Leu Met Leu Leu Arg Gln Ser
                805                 810                 815

Pro Thr Pro His Gly Leu Ser Leu Ser Asp Leu Gln Glu Ala Lys Tyr
            820                 825                 830

Glu Thr Phe Ser Asp Asp Pro Ser Pro Gly Ala Ile Asp Ser Asn Asn
        835                 840                 845

Ser Leu Ser Glu Met Thr His Phe Arg Pro Gln Leu His His Ser Gly
    850                 855                 860

Asp Met Val Phe Thr Pro Glu Ser Gly Leu Gln Leu Arg Leu Asn Glu
865                 870                 875                 880

Lys Leu Gly Thr Thr Ala Ala Thr Glu Leu Lys Lys Leu Asp Phe Lys
                885                 890                 895

Val Ser Ser Thr Ser Asn Asn Leu Ile Ser Thr Ile Pro Ser Asp Asn
            900                 905                 910

Leu Ala Ala Gly Thr Asp Asn Thr Ser Ser Leu Gly Pro Pro Ser Met
        915                 920                 925

Pro Val His Tyr Asp Ser Gln Leu Asp Thr Thr Leu Phe Gly Lys Lys
    930                 935                 940

Ser Ser Pro Leu Thr Glu Ser Gly Gly Pro Leu Ser Leu Ser Glu Glu
945                 950                 955                 960

Asn Asn Asp Ser Lys Leu Leu Glu Ser Gly Leu Met Asn Ser Gln Glu
                965                 970                 975

Ser Ser Trp Gly Lys Asn Val Ser Ser Thr Glu Ser Gly Arg Leu Phe
            980                 985                 990

Lys Gly Lys Arg Ala His Gly Pro Ala Leu Leu Thr Lys Asp Asn Ala
        995                 1000                1005

Leu Phe Lys Val Ser Ile Ser Leu Leu Lys Thr Asn Lys Thr Ser Asn
    1010                1015                1020

Asn Ser Ala Thr Asn Arg Lys Thr His Ile Asp Gly Pro Ser Leu Leu
1025                1030                1035                1040

Ile Glu Asn Ser Pro Ser Val Trp Gln Asn Ile Leu Glu Ser Asp Thr
                1045                1050                1055

Glu Phe Lys Lys Val Thr Pro Leu Ile His Asp Arg Met Leu Met Asp
            1060                1065                1070

Lys Asn Ala Thr Ala Leu Arg Leu Asn His Met Ser Asn Lys Thr Thr
        1075                1080                1085

Ser Ser Lys Asn Met Glu Met Val Gln Gln Lys Lys Glu Gly Pro Ile
    1090                1095                1100

Pro Pro Asp Ala Gln Asn Pro Asp Met Ser Phe Phe Lys Met Leu Phe
1105                1110                1115                1120

Leu Pro Glu Ser Ala Arg Trp Ile Gln Arg Thr His Gly Lys Asn Ser
                1125                1130                1135

Leu Asn Ser Gly Gln Gly Pro Ser Pro Lys Gln Leu Val Ser Leu Gly
            1140                1145                1150

Pro Glu Lys Ser Val Glu Gly Gln Asn Phe Leu Ser Glu Lys Asn Lys
        1155                1160                1165

Val Val Val Gly Lys Gly Glu Phe Thr Lys Asp Val Gly Leu Lys Glu
    1170                1175                1180

Met Val Phe Pro Ser Ser Arg Asn Leu Phe Leu Thr Asn Leu Asp Asn
1185                1190                1195                1200

Leu His Glu Asn Asn Thr His Asn Gln Glu Lys Lys Ile Gln Glu Glu
                1205                1210                1215

Ile Glu Lys Lys Glu Thr Leu Ile Gln Glu Asn Val Val Leu Pro Gln
            1220                1225                1230

Ile His Thr Val Thr Gly Thr Lys Asn Phe Met Lys Asn Leu Phe Leu
        1235                1240                1245

Leu Ser Thr Arg Gln Asn Val Glu Gly Ser Tyr Asp Gly Ala Tyr Ala
    1250                1255                1260

Pro Val Leu Gln Asp Phe Arg Ser Leu Asn Asp Ser Thr Asn Arg Thr
1265                1270                1275                1280

Lys Lys His Thr Ala His Phe Ser Lys Lys Gly Glu Glu Glu Asn Leu
                1285                1290                1295

Glu Gly Leu Gly Asn Gln Thr Lys Gln Ile Val Glu Lys Tyr Ala Cys
            1300                1305                1310

Thr Thr Arg Ile Ser Pro Asn Thr Ser Gln Gln Asn Phe Val Thr Gln
        1315                1320                1325

Arg Ser Lys Arg Ala Leu Lys Gln Phe Arg Leu Pro Leu Glu Glu Thr
    1330                1335                1340

Glu Leu Glu Lys Arg Ile Ile Val Asp Asp Thr Ser Thr Gln Trp Ser
1345                1350                1355                1360

Lys Asn Met Lys His Leu Thr Pro Ser Thr Leu Thr Gln Ile Asp Tyr
                1365                1370                1375

Asn Glu Lys Glu Lys Gly Ala Ile Thr Gln Ser Pro Leu Ser Asp Cys
            1380                1385                1390

Leu Thr Arg Ser His Ser Ile Pro Gln Ala Asn Arg Ser Pro Leu Pro
        1395                1400                1405

Ile Ala Lys Val Ser Ser Phe Pro Ser Ile Arg Pro Ile Tyr Leu Thr
    1410                1415                1420

Arg Val Leu Phe Gln Asp Asn Ser Ser His Leu Pro Ala Ala Ser Tyr
1425                1430                1435                1440

Arg Lys Lys Asp Ser Gly Val Gln Glu Ser Ser His Phe Leu Gln Gly
                1445                1450                1455

Ala Lys Lys Asn Asn Leu Ser Leu Ala Ile Leu Thr Leu Glu Met Thr
            1460                1465                1470

Gly Asp Gln Arg Glu Val Gly Ser Leu Gly Thr Ser Ala Thr Asn Ser
        1475                1480                1485

Val Thr Tyr Lys Lys Val Glu Asn Thr Val Leu Pro Lys Pro Asp Leu
    1490                1495                1500

Pro Lys Thr Ser Gly Lys Val Glu Leu Leu Pro Lys Val His Ile Tyr
1505                1510                1515                1520

Gln Lys Asp Leu Phe Pro Thr Glu Thr Ser Asn Gly Ser Pro Gly His
                1525                1530                1535

Leu Asp Leu Val Glu Gly Ser Leu Leu Gln Gly Thr Glu Gly Ala Ile
            1540                1545                1550

Lys Trp Asn Glu Ala Asn Arg Pro Gly Lys Val Pro Phe Leu Arg Val
        1555                1560                1565

Ala Thr Glu Ser Ser Ala Lys Thr Pro Ser Lys Leu Leu Asp Pro Leu
    1570                1575                1580

Ala Trp Asp Asn His Tyr Gly Thr Gln Ile Pro Lys Glu Glu Trp Lys
1585                1590                1595                1600

Ser Gln Glu Lys Ser Pro Glu Lys Thr Ala Phe Lys Lys Lys Asp Thr
                1605                1610                1615

Ile Leu Ser Leu Asn Ala Cys Glu Ser Asn His Ala Ile Ala Ala Ile
            1620                1625                1630

Asn Glu Gly Gln Asn Lys Pro Glu Ile Glu Val Thr Trp Ala Lys Gln
        1635                1640                1645

Gly Arg Thr Glu Arg Leu Cys Ser Gln Asn Pro Pro Val Leu Lys Arg
    1650                1655                1660

His Gln Arg Glu Ile Thr Arg Thr Thr Leu Gln Ser Asp Gln Glu Glu
1665                1670                1675                1680

Ile Asp Tyr Asp Asp Thr Ile Ser Val Glu Met Lys Lys Glu Asp Phe
                1685                1690                1695

Asp Ile Tyr Asp Glu Asp Glu Asn Gln Ser Pro Arg Ser Phe Gln Lys
            1700                1705                1710

Lys Thr Arg His Tyr Phe Ile Ala Ala Val Glu Arg Leu Trp Asp Tyr
        1715                1720                1725

Gly Met Ser Ser Ser Pro His Val Leu Arg Asn Arg Ala Gln Ser Gly
    1730                1735                1740

Ser Val Pro Gln Phe Lys Lys Val Val Phe Gln Glu Phe Thr Asp Gly
1745                1750                1755                1760

Ser Phe Thr Gln Pro Leu Tyr Arg Gly Glu Leu Asn Glu His Leu Gly
                1765                1770                1775

Leu Leu Gly Pro Tyr Ile Arg Ala Glu Val Glu Asp Asn Ile Met Val
            1780                1785                1790

Thr Phe Arg Asn Gln Ala Ser Arg Pro Tyr Ser Phe Tyr Ser Ser Leu
        1795                1800                1805

Ile Ser Tyr Glu Glu Asp Gln Arg Gln Gly Ala Glu Pro Arg Lys Asn
    1810                1815                1820

Phe Val Lys Pro Asn Glu Thr Lys Thr Tyr Phe Trp Lys Val Gln His
1825                1830                1835                1840

His Met Ala Pro Thr Lys Asp Glu Phe Asp Cys Lys Ala Trp Ala Tyr
                1845                1850                1855

Phe Ser Asp Val Asp Leu Glu Lys Asp Val His Ser Gly Leu Ile Gly
            1860                1865                1870

Pro Leu Leu Val Cys His Thr Asn Thr Leu Asn Pro Ala His Gly Arg
        1875                1880                1885

Gln Val Thr Val Gln Glu Phe Ala Leu Phe Phe Thr Ile Phe Asp Glu
    1890                1895                1900

Thr Lys Ser Trp Tyr Phe Thr Glu Asn Met Glu Arg Asn Cys Arg Ala
1905                1910                1915                1920

Pro Cys Asn Ile Gln Met Glu Asp Pro Thr Phe Lys Glu Asn Tyr Arg
                1925                1930                1935

Phe His Ala Ile Asn Gly Tyr Ile Met Asp Thr Leu Pro Gly Leu Val
            1940                1945                1950

Met Ala Gln Asp Gln Arg Ile Arg Trp Tyr Leu Leu Ser Met Gly Ser
        1955                1960                1965

Asn Glu Asn Ile His Ser Ile His Phe Ser Gly His Val Phe Thr Val
    1970                1975                1980

Arg Lys Lys Glu Glu Tyr Lys Met Ala Leu Tyr Asn Leu Tyr Pro Gly
1985                1990                1995                2000

Val Phe Glu Thr Val Glu Met Leu Pro Ser Lys Ala Gly Ile Trp Arg
                2005                2010                2015

Val Glu Cys Leu Ile Gly Glu His Leu His Ala Gly Met Ser Thr Leu
            2020                2025                2030

Phe Leu Val Tyr Ser Asn Lys Cys Gln Thr Pro Leu Gly Met Ala Ser
        2035                2040                2045

Gly His Ile Arg Asp Phe Gln Ile Thr Ala Ser Gly Gln Tyr Gly Gln
    2050                2055                2060

Trp Ala Pro Lys Leu Ala Arg Leu His Tyr Ser Gly Ser Ile Asn Ala
2065                2070                2075                2080

Trp Ser Thr Lys Glu Pro Phe Ser Trp Ile Lys Val Asp Leu Leu Ala
                2085                2090                2095

Pro Met Ile Ile His Gly Ile Lys Thr Gln Gly Ala Arg Gln Lys Phe
            2100                2105                2110

Ser Ser Leu Tyr Ile Ser Gln Phe Ile Ile Met Tyr Ser Leu Asp Gly
        2115                2120                2125

Lys Lys Trp Gln Thr Tyr Arg Gly Asn Ser Thr Gly Thr Leu Met Val
    2130                2135                2140

Phe Phe Gly Asn Val Asp Ser Ser Gly Ile Lys His Asn Ile Phe Asn
2145                2150                2155                2160

Pro Pro Ile Ile Ala Arg Tyr Ile Arg Leu His Pro Thr His Tyr Ser
                2165                2170                2175

Ile Arg Ser Thr Leu Arg Met Glu Leu Met Gly Cys Asp Leu Asn Ser
            2180                2185                2190

Cys Ser Met Pro Leu Gly Met Glu Ser Lys Ala Ile Ser Asp Ala Gln
        2195                2200                2205

Ile Thr Ala Ser Ser Tyr Phe Thr Asn Met Phe Ala Thr Trp Ser Pro
    2210                2215                2220

Ser Lys Ala Arg Leu His Leu Gln Gly Arg Ser Asn Ala Trp Arg Pro
2225                2230                2235                2240

Gln Val Asn Asn Pro Lys Glu Trp Leu Gln Val Asp Phe Gln Lys Thr
                2245                2250                2255

Met Lys Val Thr Gly Val Thr Thr Gln Gly Val Lys Ser Leu Leu Thr
            2260                2265                2270

Ser Met Tyr Val Lys Glu Phe Leu Ile Ser Ser Ser Gln Asp Gly His
        2275                2280                2285

Gln Trp Thr Leu Phe Phe Gln Asn Gly Lys Val Lys Val Phe Gln Gly
    2290                2295                2300

Asn Gln Asp Ser Phe Thr Pro Val Val Asn Ser Leu Asp Pro Pro Leu
2305                2310                2315                2320

Leu Thr Arg Tyr Leu Arg Ile His Pro Gln Ser Trp Val His Gln Ile
                2325                2330                2335

Ala Leu Arg Met Glu Val Leu Gly Cys Glu Ala Gln Asp Leu Tyr
            2340                2345                2350

<210>    2
<211>    7056
<212>    DNA
<213>    Artificial Sequence

<220>
<223>    nucleotide sequence of Factor 8

<400>    2
atgcaaatag agctctccac ctgcttcttt ctgtgccttt tgcgattctg ctttagtgcc      60

accagaagat actacctggg tgcagtggaa ctgtcatggg actatatgca aagtgatctc     120

ggtgagctgc ctgtggacgc aagatttcct cctagagtgc caaaatcttt tccattcaac     180

acctcagtcg tgtacaaaaa gactctgttt gtagaattca cggatcacct tttcaacatc     240

gctaagccaa ggccaccctg gatgggtctg ctaggtccta ccatccaggc tgaggtttat     300

gatacagtgg tcattacact taagaacatg gcttcccatc ctgtcagtct tcatgctgtt     360

ggtgtatcct actggaaagc ttctgaggga gctgaatatg atgatcagac cagtcaaagg     420

gagaaagaag atgataaagt cttccctggt ggaagccata catatgtctg gcaggtcctg     480

aaagagaatg gtccaatggc ctctgaccca ctgtgcctta cctactcata tctttctcat     540

gtggacctgg taaaagactt gaattcaggc ctcattggag ccctactagt atgtagagaa     600

gggagtctgg ccaaggaaaa gacacagacc ttgcacaaat ttatactact ttttgctgta     660

tttgatgaag ggaaaagttg gcactcagaa acaaagaact ccttgatgca ggatagggat     720

gctgcatctg ctcgggcctg gcctaaaatg cacacagtca atggttatgt aaacaggtct     780

ctgccaggtc tgattggatg ccacaggaaa tcagtctatt ggcatgtgat tggaatgggc 840

accactcctg aagtgcactc aatattcctc gaaggtcaca catttcttgt gaggaaccat 900

cgccaggcgt ccttggaaat ctcgccaata actttcctta ctgctcaaac actcttgatg 960

gaccttggac agtttctact gttttgtcat atctcttccc accaacatga tggcatggaa 1020

gcttatgtca aagtagacag ctgtccagag gaaccccaac tacgaatgaa aaataatgaa 1080

gaagcggaag actatgatga tgatcttact gattctgaaa tggatgtggt caggtttgat 1140

gatgacaact ctccttcctt tatccaaatt cgctcagttg ccaagaagca tcctaaaact 1200

tgggtacatt acattgctgc tgaagaggag gactgggact atgctccctt agtcctcgcc 1260

cccgatgaca gaagttataa aagtcaatat ttgaacaatg gccctcagcg gattggtagg 1320

aagtacaaaa aagtccgatt tatggcatac acagatgaaa cctttaagac tcgtgaagct 1380

attcagcatg aatcaggaat cttgggacct ttactttatg gggaagttgg agacacactg 1440

ttgattatat ttaagaatca agcaagcaga ccatataaca tctaccctca cggaatcact 1500

gatgtccgtc ctttgtattc aaggagatta ccaaaaggtg taaaacattt gaaggatttt 1560

ccaattctgc caggagaaat attcaaatat aaatggacag tgactgtaga agatgggcca 1620

actaaatcag atcctcggtg cctgacccgc tattactcta gtttcgttaa tatggagaga 1680

gatctagctt caggactcat tggccctctc ctcatctgct acaaagaatc tgtagatcaa 1740

agaggaaacc agataatgtc agacaagagg aatgtcatcc tgttttctgt atttgatgag 1800

aaccgaagct ggtacctcac agagaatata caacgctttc tccccaatcc agctggagtg 1860

cagcttgagg atccagagtt ccaagcctcc aacatcatgc acagcatcaa tggctatgtt 1920

tttgatagtt tgcagttgtc agtttgtttg catgaggtgg catactggta cattctaagc 1980

attggagcac agactgactt cctttctgtc ttcttctctg gatatacctt caaacacaaa 2040

atggtctatg aagacacact caccctattc ccattctcag gagaaactgt cttcatgtcg 2100

atggaaaacc caggtctatg gattctgggg tgccacaact cagactttcg gaacagaggc 2160

atgaccgcct tactgaaggt ttctagttgt gacaagaaca ctggtgatta ttacgaggac 2220

agttatgaag atatttcagc atacttgctg agtaaaaaca atgccattga accaagaagc 2280

ttctcccaga attcaagaca ccctagcact aggcaaaagc aatttaatgc caccacaatt 2340

ccagaaaatg acatagagaa gactgaccct tggtttgcac acagaacacc tatgcctaaa 2400

atacaaaatg tctcctctag tgatttgttg atgctcttgc gacagagtcc tactccacat 2460

gggctatcct tatctgatct ccaagaagcc aaatatgaga ctttttctga tgatccatca 2520

cctggagcaa tagacagtaa taacagcctg tctgaaatga cacacttcag gccacagctc 2580

catcacagtg gggacatggt atttacccct gagtcaggcc tccaattaag attaaatgag 2640

aaactgggga caactgcagc aacagagttg aagaaacttg atttcaaagt ttctagtaca 2700

tcaaataatc tgatttcaac aattccatca gacaatttgg cagcaggtac tgataataca 2760

agttccttag gacccccaag tatgccagtt cattatgata gtcaattaga taccactcta 2820

tttggcaaaa agtcatctcc ccttactgag tctggtggac ctctgagctt gagtgaagaa 2880

aataatgatt caaagttgtt agaatcaggt ttaatgaata gccaagaaag ttcatgggga 2940

aaaaatgtat cgtcaacaga gagtggtagg ttatttaaag ggaaaagagc tcatggacct 3000

gctttgttga ctaaagataa tgccttattc aaagttagca tctctttgtt aaagacaaac 3060

aaaacttcca ataattcagc aactaataga aagactcaca ttgatggccc atcattatta 3120

attgagaata gtccatcagt ctggcaaaat atattagaaa gtgacactga gtttaaaaaa 3180

gtgacacctt tgattcatga cagaatgctt atggacaaaa atgctacagc tttgaggcta 3240

aatcatatgt caaataaaac tacttcatca aaaaacatgg aaatggtcca acagaaaaaa 3300

gagggcccca ttccaccaga tgcacaaaat ccagatatgt cgttctttaa gatgctattc 3360

ttgccagaat cagcaaggtg gatacaaagg actcatggaa agaactctct gaactctggg 3420

caaggcccca gtccaaagca attagtatcc ttaggaccag aaaaatctgt ggaaggtcag 3480

aatttcttgt ctgagaaaaa caaagtggta gtaggaaagg gtgaatttac aaaggacgta 3540

ggactcaaag agatggtttt tccaagcagc agaaacctat ttcttactaa cttggataat 3600

ttacatgaaa ataatacaca caatcaagaa aaaaaaattc aggaagaaat agaaaagaag 3660

gaaacattaa tccaagagaa tgtagttttg cctcagatac atacagtgac tggcactaag 3720

aatttcatga agaacctttt cttactgagc actaggcaaa atgtagaagg ttcatatgac 3780

ggggcatatg ctccagtact tcaagatttt aggtcattaa atgattcaac aaatagaaca 3840

aagaaacaca cagctcattt ctcaaaaaaa ggggaggaag aaaacttgga aggcttggga 3900

aatcaaacca agcaaattgt agagaaatat gcatgcacca caaggatatc tcctaataca 3960

agccagcaga attttgtcac gcaacgtagt aagagagctt tgaaacaatt cagactccca 4020

ctagaagaaa cagaacttga aaaaaggata attgtggatg acacctcaac ccagtggtcc 4080

aaaaacatga aacatttgac cccgagcacc ctcacacaga tagactacaa tgagaaggag 4140

aaaggggcca ttactcagtc tcccttatca gattgcctta cgaggagtca tagcatccct 4200

caagcaaata gatctccatt acccattgca aaggtatcat catttccatc tattagacct 4260

atatatctga ccagggtcct attccaagac aactcttctc atcttccagc agcatcttat 4320

agaaagaaag attctggggt ccaagaaagc agtcatttct tacaaggagc caaaaaaaat 4380

aacctttctt tagccattct aaccttggag atgactggtg atcaaagaga ggttggctcc 4440

ctggggacaa gtgccacaaa ttcagtcaca tacaagaaag ttgagaacac tgttctcccg 4500

aaaccagact tgcccaaaac atctggcaaa gttgaattgc ttccaaaagt tcacatttat 4560

cagaaggacc tattccctac ggaaactagc aatgggtctc ctggccatct ggatctcgtg 4620

gaagggagcc ttcttcaggg aacagaggga gcgattaagt ggaatgaagc aaacagacct 4680

ggaaaagttc cctttctgag agtagcaaca gaaagctctg caaagactcc ctccaagcta 4740

ttggatcctc ttgcttggga taaccactat ggtactcaga taccaaaaga agagtggaaa 4800

tcccaagaga agtcaccaga aaaaacagct tttaagaaaa aggataccat tttgtccctg 4860

aacgcttgtg aaagcaatca tgcaatagca gcaataaatg agggacaaaa taagcccgaa 4920

atagaagtca cctgggcaaa gcaaggtagg actgaaaggc tgtgctctca aaacccacca 4980

gtcttgaaac gccatcaacg ggaaataact cgtactactc ttcagtcaga tcaagaggaa 5040

attgactatg atgataccat atcagttgaa atgaagaagg aagattttga catttatgat 5100

gaggatgaaa atcagagccc ccgcagcttt caaaagaaaa cacgacacta ttttattgct 5160

gcagtggaga ggctctggga ttatgggatg agtagctccc cacatgttct aagaaacagg 5220

gctcagagtg gcagtgtccc tcagttcaag aaagttgttt tccaggaatt tactgatggc 5280

tcctttactc agcccttata ccgtggagaa ctaaatgaac atttgggact cctggggcca 5340

tatataagag cagaagttga agataatatc atggtaactt tcagaaatca ggcctctcgt 5400

ccctattcct tctattctag ccttatttct tatgaggaag atcagaggca aggagcagaa 5460

cctagaaaaa actttgtcaa gcctaatgaa accaaaactt acttttggaa agtgcaacat 5520

catatggcac ccactaaaga tgagtttgac tgcaaagcct gggcttattt ctctgatgtt 5580

gacctggaaa aagatgtgca ctcaggcctg attggacccc ttctggtctg ccacactaac 5640

acactgaacc ctgctcatgg gagacaagtg acagtacagg aatttgctct gtttttcacc 5700

atctttgatg agaccaaaag ctggtacttc actgaaaata tggaaagaaa ctgcagggct 5760

ccctgcaata tccagatgga agatcccact tttaaagaga attatcgctt ccatgcaatc 5820

aatggctaca taatggatac actacctggc ttagtaatgg ctcaggatca aaggattcga 5880

tggtatctgc tcagcatggg cagcaatgaa aacatccatt ctattcattt cagtggacat 5940

gtgttcactg tacgaaaaaa agaggagtat aaaatggcac tgtacaatct ctatccaggt 6000

gtttttgaga cagtggaaat gttaccatcc aaagctggaa tttggcgggt ggaatgcctt 6060

attggcgagc atctacatgc tgggatgagc acactttttc tggtgtacag caataagtgt 6120

cagactcccc tgggaatggc ttctggacac attagagatt ttcagattac agcttcagga 6180

caatatggac agtgggcccc aaagctggcc agacttcatt attccggatc aatcaatgcc 6240

tggagcacca aggagccctt ttcttggatc aaggtggatc tgttggcacc aatgattatt 6300

cacggcatca agacccaggg tgcccgtcag aagttctcca gcctctacat ctctcagttt 6360

atcatcatgt atagtcttga tgggaagaag tggcagactt atcgaggaaa ttccactgga 6420

accttaatgg tcttctttgg caatgtggat tcatctggga taaaacacaa tatttttaac 6480

cctccaatta ttgctcgata catccgtttg cacccaactc attatagcat tcgcagcact 6540

cttcgcatgg agttgatggg ctgtgattta aatagttgca gcatgccatt gggaatggag 6600

agtaaagcaa tatcagatgc acagattact gcttcatcct actttaccaa tatgtttgcc 6660

acctggtctc cttcaaaagc tcgacttcac ctccaaggga ggagtaatgc ctggagacct 6720

caggtgaata atccaaaaga gtggctgcaa gtggacttcc agaagacaat gaaagtcaca 6780

ggagtaacta ctcagggagt aaaatctctg cttaccagca tgtatgtgaa ggagttcctc 6840

atctccagca gtcaagatgg ccatcagtgg actctctttt ttcagaatgg caaagtaaag 6900

gtttttcagg gaaatcaaga ctccttcaca cctgtggtga actctctaga cccaccgtta 6960

ctgactcgct accttcgaat tcacccccag agttgggtgc accagattgc cctgaggatg 7020

gaggttctgg gctgcgaggc acaggacctc tactga 7056

<210> 3
<211> 1463
<212> PRT
<213> Artificial Sequence

<220>
<223> amino acid sequence of Factor 8 Mutant

<400> 3
Met Gln Ile Glu Leu Ser Thr Cys Phe Phe Leu Cys Leu Leu Arg Phe
1               5                   10                  15

Cys Phe Ser Ala Thr Arg Arg Tyr Tyr Leu Gly Ala Val Glu Leu Ser
            20                  25                  30

Trp Asp Tyr Met Gln Ser Asp Leu Gly Glu Leu Pro Val Asp Ala Arg
        35                  40                  45

Phe Pro Pro Arg Val Pro Lys Ser Phe Pro Phe Asn Thr Ser Val Val
    50                  55                  60

Tyr Lys Lys Thr Leu Phe Val Glu Phe Thr Asp His Leu Phe Asn Ile
65                  70                  75                  80

Ala Lys Pro Arg Pro Pro Trp Met Gly Leu Leu Gly Pro Thr Ile Gln
                85                  90                  95

Ala Glu Val Tyr Asp Thr Val Val Ile Thr Leu Lys Asn Met Ala Ser
            100                 105                 110

His Pro Val Ser Leu His Ala Val Gly Val Ser Tyr Trp Lys Ala Ser
        115                 120                 125

Glu Gly Ala Glu Tyr Asp Asp Gln Thr Ser Gln Arg Glu Lys Glu Asp
    130                 135                 140

Asp Lys Val Phe Pro Gly Gly Ser His Thr Tyr Val Trp Gln Val Leu
145                 150                 155                 160

Lys Glu Asn Gly Pro Met Ala Ser Asp Pro Leu Cys Leu Thr Tyr Ser

165                 170                 175

Tyr Leu Ser His Val Asp Leu Val Lys Asp Leu Asn Ser Gly Leu Ile
            180                 185                 190

Gly Ala Leu Leu Val Cys Arg Glu Gly Ser Leu Ala Lys Glu Lys Thr
        195                 200                 205

Gln Thr Leu His Lys Phe Ile Leu Leu Phe Ala Val Phe Asp Glu Gly
    210                 215                 220

Lys Ser Trp His Ser Glu Thr Lys Asn Ser Leu Met Gln Asp Arg Asp
225                 230                 235                 240

Ala Ala Ser Ala Arg Ala Trp Pro Lys Met His Thr Val Asn Gly Tyr
                245                 250                 255

Val Asn Arg Ser Leu Pro Gly Leu Ile Gly Cys His Arg Lys Ser Val
            260                 265                 270

Tyr Trp His Val Ile Gly Met Gly Thr Thr Pro Glu Val His Ser Ile
        275                 280                 285

Phe Leu Glu Gly His Thr Phe Leu Val Arg Asn His Arg Gln Ala Ser
    290                 295                 300

Leu Glu Ile Ser Pro Ile Thr Phe Leu Thr Ala Gln Thr Leu Leu Met
305                 310                 315                 320

Asp Leu Gly Gln Phe Leu Leu Phe Cys His Ile Ser Ser His Gln His
                325                 330                 335

Asp Gly Met Glu Ala Tyr Val Lys Val Asp Ser Cys Pro Glu Glu Pro
            340                 345                 350

Gln Leu Arg Met Lys Asn Asn Glu Glu Ala Glu Asp Tyr Asp Asp Asp
        355                 360                 365

Leu Thr Asp Ser Glu Met Asp Val Val Arg Phe Asp Asp Asp Asn Ser
    370                 375                 380

Pro Ser Phe Ile Gln Ile Arg Ser Val Ala Lys Lys His Pro Lys Thr
385                 390                 395                 400

Trp Val His Tyr Ile Ala Ala Glu Glu Glu Asp Trp Asp Tyr Ala Pro
                405                 410                 415

Leu Val Leu Ala Pro Asp Asp Arg Ser Tyr Lys Ser Gln Tyr Leu Asn
            420                 425                 430

Asn Gly Pro Gln Arg Ile Gly Arg Lys Tyr Lys Lys Val Arg Phe Met
        435                 440                 445

Ala Tyr Thr Asp Glu Thr Phe Lys Thr Arg Glu Ala Ile Gln His Glu
    450                 455                 460

Ser Gly Ile Leu Gly Pro Leu Leu Tyr Gly Glu Val Gly Asp Thr Leu
465                 470                 475                 480

Leu Ile Ile Phe Lys Asn Gln Ala Ser Arg Pro Tyr Asn Ile Tyr Pro
                485                 490                 495

His Gly Ile Thr Asp Val Arg Pro Leu Tyr Ser Arg Arg Leu Pro Lys

500                 505                 510

Gly Val Lys His Leu Lys Asp Phe Pro Ile Leu Pro Gly Glu Ile Phe
        515                 520                 525

Lys Tyr Lys Trp Thr Val Thr Val Glu Asp Gly Pro Thr Lys Ser Asp
    530                 535                 540

Pro Arg Cys Leu Thr Arg Tyr Tyr Ser Ser Phe Val Asn Met Glu Arg
545                 550                 555                 560

Asp Leu Ala Ser Gly Leu Ile Gly Pro Leu Leu Ile Cys Tyr Lys Glu
                565                 570                 575

Ser Val Asp Gln Arg Gly Asn Gln Ile Met Ser Asp Lys Arg Asn Val
            580                 585                 590

Ile Leu Phe Ser Val Phe Asp Glu Asn Arg Ser Trp Tyr Leu Thr Glu
        595                 600                 605

Asn Ile Gln Arg Phe Leu Pro Asn Pro Ala Gly Val Gln Leu Glu Asp
    610                 615                 620

Pro Glu Phe Gln Ala Ser Asn Ile Met His Ser Ile Asn Gly Tyr Val
625                 630                 635                 640

Phe Asp Ser Leu Gln Leu Ser Val Cys Leu His Glu Val Ala Tyr Trp
                645                 650                 655

Tyr Ile Leu Ser Ile Gly Ala Gln Thr Asp Phe Leu Ser Val Phe Phe
            660                 665                 670

Ser Gly Tyr Thr Phe Lys His Lys Met Val Tyr Glu Asp Thr Leu Thr
        675                 680                 685

Leu Phe Pro Phe Ser Gly Glu Thr Val Phe Met Ser Met Glu Asn Pro
    690                 695                 700

Gly Leu Trp Ile Leu Gly Cys His Asn Ser Asp Phe Arg Asn Arg Gly
705                 710                 715                 720

Met Thr Ala Leu Leu Lys Val Ser Ser Cys Asp Lys Asn Thr Gly Asp
                725                 730                 735

Tyr Tyr Glu Asp Ser Tyr Glu Asp Ile Ser Ala Tyr Leu Leu Ser Lys
            740                 745                 750

Asn Asn Ala Ile Glu Pro Arg Ser Phe Ser Gln Asn Ser Arg His Pro
        755                 760                 765

Ser Thr Arg Gln Lys Gln Phe Asn Ala Thr Thr Ile Pro Glu Asn Thr
    770                 775                 780

Thr Leu Gln Ser Asp Gln Glu Glu Ile Asp Tyr Asp Asp Thr Ile Ser
785                 790                 795                 800

Val Glu Met Lys Lys Glu Asp Phe Asp Ile Tyr Asp Glu Asp Glu Asn
                805                 810                 815

Gln Ser Pro Arg Ser Phe Gln Lys Lys Thr Arg His Tyr Phe Ile Ala
            820                 825                 830

Ala Val Glu Arg Leu Trp Asp Tyr Gly Met Ser Ser Ser Pro His Val

835                 840                 845

Leu Arg Asn Arg Ala Gln Ser Gly Ser Val Pro Gln Phe Lys Lys Val
    850                 855                 860

Val Phe Gln Glu Phe Thr Asp Gly Ser Phe Thr Gln Pro Leu Tyr Arg
865                 870                 875                 880

Gly Glu Leu Asn Glu His Leu Gly Leu Leu Gly Pro Tyr Ile Arg Ala
                885                 890                 895

Glu Val Glu Asp Asn Ile Met Val Thr Phe Arg Asn Gln Ala Ser Arg
            900                 905                 910

Pro Tyr Ser Phe Tyr Ser Ser Leu Ile Ser Tyr Glu Glu Asp Gln Arg
        915                 920                 925

Gln Gly Ala Glu Pro Arg Lys Asn Phe Val Lys Pro Asn Glu Thr Lys
    930                 935                 940

Thr Tyr Phe Trp Lys Val Gln His His Met Ala Pro Thr Lys Asp Glu
945                 950                 955                 960

Phe Asp Cys Lys Ala Trp Ala Tyr Phe Ser Asp Val Asp Leu Glu Lys
                965                 970                 975

Asp Val His Ser Gly Leu Ile Gly Pro Leu Leu Val Cys His Thr Asn
            980                 985                 990

Thr Leu Asn Pro Ala His Gly Arg Gln Val Thr Val Gln Glu Phe Ala
        995                 1000                1005

Leu Phe Phe Thr Ile Phe Asp Glu Thr Lys Ser Trp Tyr Phe Thr Glu
    1010                1015                1020

Asn Met Glu Arg Asn Cys Arg Ala Pro Cys Asn Ile Gln Met Glu Asp
1025                1030                1035                1040

Pro Thr Phe Lys Glu Asn Tyr Arg Phe His Ala Ile Asn Gly Tyr Ile
                1045                1050                1055

Met Asp Thr Leu Pro Gly Leu Val Met Ala Gln Asp Gln Arg Ile Arg
            1060                1065                1070

Trp Tyr Leu Leu Ser Met Gly Ser Asn Glu Asn Ile His Ser Ile His
        1075                1080                1085

Phe Ser Gly His Val Phe Thr Val Arg Lys Lys Glu Glu Tyr Lys Met
    1090                1095                1100

Ala Leu Tyr Asn Leu Tyr Pro Gly Val Phe Glu Thr Val Glu Met Leu
1105                1110                1115                1120

Pro Ser Lys Ala Gly Ile Trp Arg Val Glu Cys Leu Ile Gly Glu His
                1125                1130                1135

Leu His Ala Gly Met Ser Thr Leu Phe Leu Val Tyr Ser Asn Lys Cys
            1140                1145                1150

Gln Thr Pro Leu Gly Met Ala Ser Gly His Ile Arg Asp Phe Gln Ile
        1155                1160                1165

Thr Ala Ser Gly Gln Tyr Gly Gln Trp Ala Pro Lys Leu Ala Arg Leu

1170                1175                1180

His Tyr Ser Gly Ser Ile Asn Ala Trp Ser Thr Lys Glu Pro Phe Ser
1185                1190                1195                1200

Trp Ile Lys Val Asp Leu Leu Ala Pro Met Ile Ile His Gly Ile Lys
                1205                1210                1215

Thr Gln Gly Ala Arg Gln Lys Phe Ser Ser Leu Tyr Ile Ser Gln Phe
            1220                1225                1230

Ile Ile Met Tyr Ser Leu Asp Gly Lys Lys Trp Gln Thr Tyr Arg Gly
        1235                1240                1245

Asn Ser Thr Gly Thr Leu Met Val Phe Phe Gly Asn Val Asp Ser Ser
    1250                1255                1260

Gly Ile Lys His Asn Ile Phe Asn Pro Pro Ile Ile Ala Arg Tyr Ile
1265                1270                1275                1280

Arg Leu His Pro Thr His Tyr Ser Ile Arg Ser Thr Leu Arg Met Glu
                1285                1290                1295

Leu Met Gly Cys Asp Leu Asn Ser Cys Ser Met Pro Leu Gly Met Glu
            1300                1305                1310

Ser Lys Ala Ile Ser Asp Ala Gln Ile Thr Ala Ser Ser Tyr Phe Thr
        1315                1320                1325

Asn Met Phe Ala Thr Trp Ser Pro Ser Lys Ala Arg Leu His Leu Gln
    1330                1335                1340

Gly Arg Ser Asn Ala Trp Arg Pro Gln Val Asn Asn Pro Lys Glu Trp
1345                1350                1355                1360

Leu Gln Val Asp Phe Gln Lys Thr Met Lys Val Thr Gly Val Thr Thr
                1365                1370                1375

Gln Gly Val Lys Ser Leu Leu Thr Ser Met Tyr Val Lys Glu Phe Leu
            1380                1385                1390

Ile Ser Ser Ser Gln Asp Gly His Gln Trp Thr Leu Phe Phe Gln Asn
        1395                1400                1405

Gly Lys Val Lys Val Phe Gln Gly Asn Gln Asp Ser Phe Thr Pro Val
    1410                1415                1420

Val Asn Ser Leu Asp Pro Pro Leu Leu Thr Arg Tyr Leu Arg Ile His
1425                1430                1435                1440

Pro Gln Ser Trp Val His Gln Ile Ala Leu Arg Met Glu Val Leu Gly
                1445                1450                1455

Cys Glu Ala Gln Asp Leu Tyr
            1460


<210>   4
<211>   2366
<212>   DNA
<213>   Artificial Sequence

<220>

<223>    Fragment 1 for factor 8 mutant

<400>    4
atgcaaatag agctctccac ctgcttcttt ctgtgccttt tgcgattctg ctttagtgcc      60

accagaagat actacctggg tgcagtggaa ctgtcatggg actatatgca aagtgatctc     120

ggtgagctgc ctgtggacgc aagatttcct cctagagtgc caaaatcttt tccattcaac     180

acctcagtcg tgtacaaaaa gactctgttt gtagaattca cggatcacct tttcaacatc     240

gctaagccaa ggccaccctg gatgggtctg ctaggtccta ccatccaggc tgaggtttat     300

gatacagtgg tcattacact taagaacatg gcttcccatc ctgtcagtct tcatgctgtt     360

ggtgtatcct actggaaagc ttctgaggga gctgaatatg atgatcagac cagtcaaagg     420

gagaaagaag atgataaagt cttccctggt ggaagccata catatgtctg gcaggtcctg     480

aaagagaatg gtccaatggc ctctgaccca ctgtgcctta cctactcata tctttctcat     540

gtggacctgg taaaagactt gaattcaggc ctcattggag ccctactagt atgtagagaa     600

gggagtctgg ccaaggaaaa gacacagacc ttgcacaaat ttatactact ttttgctgta     660

tttgatgaag ggaaaagttg gcactcagaa acaaagaact ccttgatgca ggatagggat     720

gctgcatctg ctcgggcctg gcctaaaatg cacacagtca atggttatgt aaacaggtct     780

ctgccaggtc tgattggatg ccacaggaaa tcagtctatt ggcatgtgat tggaatgggc     840

accactcctg aagtgcactc aatattcctc gaaggtcaca catttcttgt gaggaaccat     900

cgccaggcgt ccttggaaat ctcgccaata actttcctta ctgctcaaac actcttgatg     960

gaccttggac agtttctact gttttgtcat atctcttccc accaacatga tggcatggaa    1020

gcttatgtca aagtagacag ctgtccagag gaaccccaac tacgaatgaa aaataatgaa    1080

gaagcggaag actatgatga tgatcttact gattctgaaa tggatgtggt caggtttgat    1140

gatgacaact ctccttcctt tatccaaatt cgctcagttg ccaagaagca tcctaaaact    1200

tgggtacatt acattgctgc tgaagaggag gactgggact atgctccctt agtcctcgcc    1260

cccgatgaca gaagttataa aagtcaatat ttgaacaatg gccctcagcg gattggtagg    1320

aagtacaaaa aagtccgatt tatggcatac acagatgaaa cctttaagac tcgtgaagct    1380

attcagcatg aatcaggaat cttgggacct ttactttatg gggaagttgg agacacactg    1440

ttgattatat ttaagaatca agcaagcaga ccatataaca tctaccctca cggaatcact    1500

gatgtccgtc ctttgtattc aaggagatta ccaaaaggtg taaaacattt gaaggatttt    1560

ccaattctgc caggagaaat attcaaatat aaatggacag tgactgtaga agatgggcca    1620

actaaatcag atcctcggtg cctgacccgc tattactcta gtttcgttaa tatggagaga    1680

gatctagctt caggactcat tggccctctc ctcatctgct acaaagaatc tgtagatcaa    1740

agaggaaacc agataatgtc agacaagagg aatgtcatcc tgttttctgt atttgatgag    1800

aaccgaagct ggtacctcac agagaatata caacgctttc tccccaatcc agctggagtg 1860

cagcttgagg atccagagtt ccaagcctcc aacatcatgc acagcatcaa tggctatgtt 1920

tttgatagtt tgcagttgtc agtttgtttg catgaggtgg catactggta cattctaagc 1980

attggagcac agactgactt cctttctgtc ttcttctctg gatatacctt caaacacaaa 2040

atggtctatg aagacacact caccctattc ccattctcag gagaaactgt cttcatgtcg 2100

atggaaaacc caggtctatg gattctgggg tgccacaact cagactttcg gaacagaggc 2160

atgaccgcct tactgaaggt ttctagttgt gacaagaaca ctggtgatta ttacgaggac 2220

agttatgaag atatttcagc atacttgctg agtaaaaaca atgccattga accaagaagc 2280

ttctcccaga attcaagaca ccctagcact aggcaaaagc aatttaatgc caccacaatt 2340

ccagaaaata ctactcttca gtcaga 2366

<210> 5
<211> 2060
<212> DNA
<213> Artificial Sequence

<220>
<223> Fragment 2 for factor 8 mutant

<400> 5
   ccacaattcc agaaaatact actcttcagt cagatcaaga ggaaattgac tatgatgata 60
   ccatatcagt tgaaatgaag aaggaagatt ttgacattta tgatgaggat gaaaatcaga 120
   gcccccgcag ctttcaaaag aaaacacgac actattttat tgctgcagtg gagaggctct 180
   gggattatgg gatgagtagc tccccacatg ttctaagaaa cagggctcag agtggcagtg 240
   tccctcagtt caagaaagtt gttttccagg aatttactga tggctccttt actcagccct 300
   tataccgtgg agaactaaat gaacatttgg gactcctggg gccatatata agagcagaag 360
   ttgaagataa tatcatggta actttcagaa atcaggcctc tcgtccctat tccttctatt 420
   ctagccttat ttcttatgag gaagatcaga ggcaaggagc agaacctaga aaaaactttg 480
   tcaagcctaa tgaaaccaaa acttactttt ggaaagtgca acatcatatg gcacccacta 540
   aagatgagtt tgactgcaaa gcctgggctt atttctctga tgttgacctg gaaaaagatg 600
   tgcactcagg cctgattgga ccccttctgg tctgccacac taacacactg aaccctgctc 660
   atgggagaca agtgacagta caggaatttg ctctgttttt caccatcttt gatgagacca 720
   aaagctggta cttcactgaa aatatggaaa gaaactgcag ggctccctgc aatatccaga 780
   tggaagatcc cacttttaaa gagaattatc gcttccatgc aatcaatggc tacataatgg 840
   atacactacc tggcttagta atggctcagg atcaaaggat tcgatggtat ctgctcagca 900
   tgggcagcaa tgaaaacatc cattctattc atttcagtgg acatgtgttc actgtacgaa 960
   aaaaagagga gtataaaatg gcactgtaca atctctatcc aggtgttttt gagacagtgg 1020
   aaatgttacc atccaaagct ggaatttggc gggtggaatg ccttattggc gagcatctac 1080
   atgctgggat gagcacactt tttctggtgt acagcaataa gtgtcagact cccctgggaa 1140
   tggcttctgg acacattaga gattttcaga ttacagcttc aggacaatat ggacagtggg 1200
   ccccaaagct ggccagactt cattattccg gatcaatcaa tgcctggagc accaaggagc 1260
   ccttttcttg gatcaaggtg gatctgttgg caccaatgat tattcacggc atcaagaccc 1320
   agggtgcccg tcagaagttc tccagcctct acatctctca gtttatcatc atgtatagtc 1380
   ttgatgggaa gaagtggcag acttatcgag gaaattccac tggaacctta atggtcttct 1440
   ttggcaatgt ggattcatct gggataaaac acaatatttt taaccctcca attattgctc 1500
   gatacatccg tttgcaccca actcattata gcattcgcag cactcttcgc atggagttga 1560
   tgggctgtga tttaaatagt tgcagcatgc cattgggaat ggagagtaaa gcaatatcag 1620
   atgcacagat tactgcttca tcctacttta ccaatatgtt tgccacctgg tctccttcaa 1680
   aagctcgact tcacctccaa gggaggagta atgcctggag acctcaggtg aataatccaa 1740
   aagagtggct gcaagtggac ttccagaaga caatgaaagt cacaggagta actactcagg 1800
   gagtaaaatc tctgcttacc agcatgtatg tgaaggagtt cctcatctcc agcagtcaag 1860
   atggccatca gtggactctc ttttttcaga atggcaaagt aaaggttttt cagggaaatc 1920
   aagactcctt cacacctgtg gtgaactctc tagacccacc gttactgact cgctaccttc 1980
   gaattcaccc ccagagttgg gtgcaccaga ttgccctgag gatggaggtt ctgggctgcg 2040
   aggcacagga cctctactga 2060

<210> 6
<211> 1386
<212> DNA
<213> Artificial Sequence

<220>
<223> nucleotide sequence of Factor 9

<400> 6
atgcagcgcg tgaacatgat catggcagaa tcaccaggcc tcatcaccat ctgcctttta 60

ggatatctac tcagtgctga atgtacagtt tttcttgatc atgaaaacgc caacaaaatt 120

ctgaatcggc caaagaggta taattcaggt aaattggaag agtttgttca agggaacctt 180

gagagagaat gtatggaaga aaagtgtagt tttgaagaag cacgagaagt ttttgaaaac 240

actgaaagaa caactgaatt ttggaagcag tatgttgatg gagatcagtg tgagtccaat 300

ccatgtttaa atggcggcag ttgcaaggat gacattaatt cctatgaatg ttggtgtccc 360

tttggatttg aaggaaagaa ctgtgaatta gatgtaacat gtaacattaa gaatggcaga 420

tgcgagcagt tttgtaaaaa tagtgctgat aacaaggtgg tttgctcctg tactgaggga 480

tatcgacttg cagaaaacca gaagtcctgt gaaccagcag tgccatttcc atgtggaaga 540

gtttctgttt cacaaacttc taagctcacc cgtgctgaga ctgtttttcc tgatgtggac 600

tatgtaaatt ctactgaagc tgaaaccatt ttggataaca tcactcaaag cacccaatca 660

tttaatgact tcactcgggt tgttggtgga gaagatgcca aaccaggtca attcccttgg 720

caggttgttt tgaatggtaa agttgatgca ttctgtggag gctctatcgt taatgaaaaa 780

tggattgtaa ctgctgccca ctgtgttgaa actggtgtta aaattacagt tgtcgcaggt 840

gaacataata ttgaggagac agaacataca gagcaaaagc gaaatgtgat tcgaattatt 900

cctcaccaca actacaatgc agctattaat aagtacaacc atgacattgc ccttctggaa 960

ctggacgaac ccttagtgct aaacagctac gttacaccta tttgcattgc tgacaaggaa 1020

tacacgaaca tcttcctcaa atttggatct ggctatgtaa gtggctgggg aagagtcttc 1080

cacaaaggga gatcagcttt agttcttcag taccttagag ttccacttgt tgaccgagcc 1140

acatgtcttc gatctacaaa gttcaccatc tataacaaca tgttctgtgc tggcttccat 1200

gaaggaggta gagattcatg tcaaggagat agtgggggac cccatgttac tgaagtggaa 1260

gggaccagtt tcttaactgg aattattagc tggggtgaag agtgtgcaat gaaaggcaaa 1320

tatggaatat ataccaaggt atcccggtat gtcaactgga ttaaggaaaa aacaaagctc 1380

acttaa 1386

<210> 7
<211> 3725
<212> DNA
<213> Artificial Sequence

<220>
<223> nucleotide sequence of pGP

<400> 7
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga ctctataggc acaccccttt ggctcttatg catgctatac 780
   tgtttttggc ttggggccta tacacccccg cttccttatg ctataggtga tggtatagct 840
   tagcctatag gtgtgggtta ttgaccatta ttgaccactc ccctattggt gacgatactt 900
   tccattacta atccataaca tggctctttg ccacaactat ctctattggc tatatgccaa 960
   tactctgtcc ttcagagact gacacggact ctgtattttt acaggatggg gtcccattta 1020
   ttatttacaa attcacatat acaacaacgc cgtcccccgt gcccgcagtt tttattaaac 1080
   atagcgtggg atctccacgc gaatctcggg tacgtgttcc ggacatgggc tcttctccgg 1140
   tagcggcgga gcttccacat ccgagccctg gtcccatgcc tccagcggct catggtcgct 1200
   cggcagctcc ttgctcctaa cagtggaggc cagacttagg cacagcacaa tgcccaccac 1260
   caccagtgtg ccgcacaagg ccgtggcggt agggtatgtg tctgaaaatg agctcggaga 1320
   ttgggctcgc accgctgacg cagatggaag acttaaggca gcggcagaag aagatgcagg 1380
   cagctgagtt gttgtattct gataagagtc agaggtaact cccgttgcgg tgctgttaac 1440
   ggtggagggc agtgtagtct gagcagtact cgttgctgcc gcgcgcgcca ccagacataa 1500
   tagctgacag actaacagac tgttcctttc catgggtctt ttctgcagtc accgtccttg 1560
   acacgaagct tgctagcgga tccgtcgacg aattcacgcg tctcgaggcg gccgctctag 1620
   agggcccgtt taaacccgct gatcagcctc gactgtgcct tctagttgcc agccatctgt 1680
   tgtttgcccc tcccccgtgc cttccttgac cctggaaggt gccactccca ctgtcctttc 1740
   ctaataaaat gaggaaattg catcgcattg tctgagtagg tgtcattcta ttctgggggg 1800
   tggggtgggg caggacagca agggggagga ttgggaagac aatagcaggc atgctgggga 1860
   gtcgaaattc agaagaactc gtcaagaagg cgatagaagg cgatgcgctg cgaatcggga 1920
   gcggcgatac cgtaaagcac gaggaagcgg tcagcccatt cgccgccaag ctcttcagca 1980
   atatcacggg tagccaacgc tatgtcctga tagcggtccg ccacacccag ccggccacag 2040
   tcgatgaatc cagaaaagcg gccattttcc accatgatat tcggcaagca ggcatcgcca 2100
   tgggtcacga cgagatcctc gccgtcgggc atgctcgcct tgagcctggc gaacagttcg 2160
   gctggcgcga gcccctgatg ctcttcgtcc agatcatcct gatcgacaag accggcttcc 2220
   atccgagtac gtgctcgctc gatgcgatgt ttcgcttggt ggtcgaatgg gcaggtagcc 2280
   ggatcaagcg tatgcagccg ccgcattgca tcagccatga tggatacttt ctcggcagga 2340
   gcaaggtgag atgacaggag atcctgcccc ggcacttcgc ccaatagcag ccagtccctt 2400
   cccgcttcag tgacaacgtc gagcacagct gcgcaaggaa cgcccgtcgt ggccagccac 2460
   gatagccgcg ctgcctcgtc ttgcagttca ttcagggcac cggacaggtc ggtcttgaca 2520
   aaaagaaccg ggcgcccctg cgctgacagc cggaacacgg cggcatcaga gcagccgatt 2580
   gtctgttgtg cccagtcata gccgaatagc ctctccaccc aagcggccgg agaacctgcg 2640
   tgcaatccat cttgttcaat catgcgaaac gatcctcatc ctgtctcttg atcagatctt 2700
   gatcccctgc gccatcagat ccttggcggc gagaaagcca tccagtttac tttgcagggc 2760
   ttcccaactt accagagggc gccccagctg gcaattccgg ttcgcttgct gtccataaaa 2820
   ccgcccagtc tagctatcgc catgtaagcc cactgcaagc tacctgcttt ctctttgcgc 2880
   ttgcgttttc ccttgtccag atagcccagt agctgacatt catccggggt cagcaccgtt 2940
   tctgcggact ggctttctac gtgaaaagga tctaggtgaa gatccttttt gataatctca 3000
   tgaccaaaat cccttaacgt gagttttcgt tccactgagc gtcagacccc gtagaaaaga 3060
   tcaaaggatc ttcttgagat cctttttttc tgcgcgtaat ctgctgcttg caaacaaaaa 3120
   aaccaccgct accagcggtg gtttgtttgc cggatcaaga gctaccaact ctttttccga 3180
   aggtaactgg cttcagcaga gcgcagatac caaatactgt ccttctagtg tagccgtagt 3240
   taggccacca cttcaagaac tctgtagcac cgcctacata cctcgctctg ctaatcctgt 3300
   taccagtggc tgctgccagt ggcgataagt cgtgtcttac cgggttggac tcaagacgat 3360
   agttaccgga taaggcgcag cggtcgggct gaacgggggg ttcgtgcaca cagcccagct 3420
   tggagcgaac gacctacacc gaactgagat acctacagcg tgagctatga gaaagcgcca 3480
   cgcttcccga agggagaaag gcggacaggt atccggtaag cggcagggtc ggaacaggag 3540
   agcgcacgag ggagcttcca gggggaaacg cctggtatct ttatagtcct gtcgggtttc 3600
   gccacctctg acttgagcgt cgatttttgt gatgctcgtc aggggggcgg agcctatgga 3660
   aaaacgccag caacgcggcc tttttacggt tcctggcctt ttgctggcct tttgctcaca 3720
   tgcgc 3725